# EUROPÄISCHE PATENTANMELDUNG

(11) **EP 3 530 754 A1**
(43) Veröffentlichungstag der Anmeldung: **28.08.2019**
(21) Anmeldenummer: 18195312.6
(22) Anmeldetag: 18.09.2018
(51) Int. Cl.: C12Q 1/6848

(54) **VERFAHREN ZUR AMPLIFIKATION EINER NUKLEINSÄURE MIT VERBESSERTER SPEZIFITÄT**

(30) Priorität: 26.02.2018 EP 18158722
(71) Anmelder: AGCT GmbH, 23562 Luebeck (DE)
(72) Erfinder: Die Erfindernennung liegt noch nicht vor
(74) Vertreter: Schulz Junghans Patentanwälte PartGmbB

(57) **Zusammenfassung**

Die vorliegende Erfindung betrifft ein Verfahren zur Amplifikation einer Nukleinsäure mit verbesserter Spezifität unter Verwendung besonderer Primer-Oligonukleotide und Controller-Nukleotide, wobei in einer ersten Amplifikation die Controller-Oligonukleotide in sequenzspezifischer Weise eine Strangöffnung im Amplifikat ermöglichen. Die Erfindung betrifft weiterhin ein entsprechendes Kit zur Durchführung des erfindungsgemäßen Verfahrens.

SEQUENCE LISTING

<110>  AGCT GmbH

<120>  Verfahren zur Amplifikation einer Nukleinsäure mit verbesserter
       Spezifität

<130>  agc105ep-1

<160>  23

<170>  PatentIn version 3.5

<210>  1
<211>  38
<212>  DNA
<213>  Homo sapiens


<220>
<221>  misc_feature
<222>  (1)..(11)
<223>  Primerbindungsstelle für ersten Primer

<220>
<221>  misc_feature
<222>  (27)..(38)
<223>  Primerbindungsstelle für zweiten Primer

<400>  1
gtaagagcag atccctggac aggcaaggaa tacaggta                              38


<210>  2
<211>  57
<212>  DNA
<213>  Artificial Sequence

<220>
<223>  primer for PCR


<220>
<221>  misc_feature
<222>  (33)..(44)
<223>  2´-O-Methyl-Modifikation

<220>
<221>  modified_base
<222>  (35)..(35)
<223>  Uracil instead of thymidine

<220>
<221>  modified_base
<222>  (37)..(37)
<223>  Uracil instead of thymidine

<220>
<221>  modified_base
<222>  (39)..(39)
<223>  Uracil instead of thymidine

<220>

<221>  modified_base
<222>  (43)..(43)
<223>  Uracil instead of thymidine

<220>
<221>  misc_feature
<222>  (44)..(45)
<223>  Nukeotid 44 ist mit Nukleotid 45 über einen C3-Linker verbunden
       und dient der Terminierung der Synthese des zweiten
       Primerverlängerungsprodukts

<220>
<221>  modified_base
<222>  (44)..(44)
<223>  Uracil instead of thymidine

<220>
<221>  misc_feature
<222>  (45)..(57)
<223>  Segment 45 bis 57 dient als Primer

<400>  2
aactcagaca agatgtgatt tttttacctg tatctctgat gcttctacct gtattcc          57


<210>  3
<211>  70
<212>  DNA
<213>  Artificial Sequence

<220>
<223>  primer für PCR


<220>
<221>  misc_feature
<222>  (35)..(36)
<223>  Nukeotid 35 ist mit Nukleotid 36 über einen HEG-Linker verbunden
       und dient der Terminierung der Synthese des
       Primerverlängerungsprodukts

<220>
<221>  misc_feature
<222>  (36)..(70)
<223>  Nukleotid 36 bis 70 dient als Primer

<400>  3
ctacagaact cagacaagat gtgaactaca atgttgctca tactacaatg tcacttactg      60

taagagcaga                                                              70


<210>  4
<211>  56
<212>  DNA
<213>  Artificial Sequence

<220>
<223>  Controller-Oligonukleotid


<220>
<221>  modified_base

<222>  (1)..(1)
<223>  Uracil instead of thymidine

<220>
<221>  misc_feature
<222>  (1)..(39)
<223>  2´-O-Me-Nukleotid-Modifikationen

<220>
<221>  modified_base
<222>  (4)..(4)
<223>  Uracil instead of thymidine

<220>
<221>  modified_base
<222>  (6)..(6)
<223>  Uracil instead of thymidine

<220>
<221>  modified_base
<222>  (8)..(8)
<223>  Uracil instead of thymidine

<220>
<221>  modified_base
<222>  (18)..(18)
<223>  Uracil instead of thymidine

<220>
<221>  modified_base
<222>  (22)..(22)
<223>  Uracil instead of thymidine

<220>
<221>  modified_base
<222>  (37)..(37)
<223>  Uracil instead of thymidine

<220>
<221>  misc_feature
<222>  (56)..(57)
<223>  3´-Phosphat-Gruppe zur Blockade einer möglichen Verlängerung
       durch Polymerase

<400>  4
taatctgtaa gagcagatcc ctggacaggc aaggaataca ggtagaagca tcagag          56


<210>  5
<211>  52
<212>  DNA
<213>  Artificial Sequence

<220>
<223>  Primer für PCR

<400>  5
aagctcgaca aaagaactca gagtgtgctc gacactacct gtattccttg cc              52


<210>  6
<211>  67
<212>  DNA

<213>  Homo sapiens



<220>
<221>  misc_feature
<222>  (1)..(35)
<223>  Das zweite Primer-Oligonukleotid bindet an das Reverse-Complement

<220>
<221>  misc_feature
<222>  (50)..(62)
<223>  Bindungssequenz für das erste Primer-Oligonukleotid

<400>  6
gctcatacta caatgtcact tactgtaaga gcagatccct ggacaggcaa ggaatacagg      60

taaaaaa                                                                67


<210>  7
<211>  67
<212>  DNA
<213>  Homo sapiens



<220>
<221>  misc_feature
<222>  (1)..(35)
<223>  Das zweite Primer-Oligonukleotid bindet an das Reverse-Complement

<220>
<221>  misc_feature
<222>  (50)..(62)
<223>  Bindungssequenz für das erste Primer-Oligonukleotid

<400>  7
gctcatacta caatgtcact tactgtaaga gcagatccct ggacaggcga ggaatacagg      60

taaaaaa                                                                67


<210>  8
<211>  70
<212>  DNA
<213>  Artificial Sequence

<220>
<223>  Primer für PCR



<220>
<221>  misc_feature
<222>  (35)..(36)
<223>  Nukeotid 35 ist mit Nukleotid 36 über einen HEG-Linker verbunden
       und dient der Terminierung der Synthese des zweiten
       Primerverlängerungsprodukts

<220>
<221>  misc_feature
<222>  (36)..(70)
<223>  Segemnt 36 bis 70 dient als Primer

<400>  8

ctacagaact cagacaagat gtgaactaca atgttgctca tactacaatg tcacttactg 60

taagagcaga 70

<210> 9
<211> 55
<212> DNA
<213> Artificial Sequence

<220>
<223> Primer für PCR

<220>
<221> misc_feature
<222> (32)..(43)
<223> O-Me-Modifikationen

<220>
<221> modified_base
<222> (33)..(33)
<223> Uracil instead of thymidine

<220>
<221> modified_base
<222> (35)..(35)
<223> Uracil instead of thymidine

<220>
<221> modified_base
<222> (38)..(38)
<223> Uracil instead of thymidine

<220>
<221> modified_base
<222> (41)..(41)
<223> Uracil instead of thymidine

<220>
<221> modified_base
<222> (42)..(42)
<223> Uracil instead of thymidine

<220>
<221> misc_feature
<222> (43)..(44)
<223> Nukleotid 43 ist mit Nukleotid 44 über einen C3-Linker verbunden
und dient zur Terminierung der Synthese des zweiten
Primer-Verlängerungsproduktes

<220>
<221> misc_feature
<222> (44)..(55)
<223> Segment 44 bis 55 dient als Primer

<400> 9
cacttacgac acaagatttt tttacctgta tctctgatgc ttctacctgt attcc 55

<210> 10
<211> 62
<212> DNA

<213>  Artificial Sequence

<220>
<223>  zweite Primer-Verlängerungsprodukt

<400>  10
gctcatacta caatgtcact tactgtaaga gcagatccct ggacaggcaa ggaatacagg      60

ta                                                                      62


<210>  11
<211>  47
<212>  DNA
<213>  Artificial Sequence

<220>
<223>  Primer für PCR


<220>
<221>  misc_feature
<222>  (47)..(47)
<223>  Tetramethylrhodamine ist am 5'-Ende des Stranges angehängt

<400>  11
acgaagctcg caagaactca gagtgtgctc gacactacct gtattcc                     47


<210>  12
<211>  23
<212>  DNA
<213>  Artificial Sequence

<220>
<223>  Primer für PCR


<220>
<221>  misc_feature
<222>  (23)..(23)
<223>  Tetramethylrhodamine ist am 5'-Ende des Stranges angehängt

<400>  12
gctcatacta caatgtcact tac                                               23


<210>  13
<211>  62
<212>  DNA
<213>  Artificial Sequence

<220>
<223>  zweites Primer-Verlängerungsprodukt der ersten Amplifikation


<220>
<221>  misc_feature
<222>  (1)..(23)
<223>  Bindungssequenz für das erste Primer-Oligonukleotid

<220>
<221>  misc_feature

<222>   (50)..(62)
<223>   Das zweite Primer-Oligonukleotid bindet an das Reverse-Complement
        der Nukleotide 50 bis 62

<400>   13
gctcatacta caatgtcact tactgtaaga gcagatccct ggacaggcaa ggaatacagg      60

ta                                                                      62


<210>   14
<211>   53
<212>   DNA
<213>   Artificial Sequence

<220>
<223>   Primer für PCR


<220>
<221>   misc_feature
<222>   (53)..(53)
<223>   Tetramethylrhodamine ist am 5'-Ende des Stranges angehängt

<400>   14
gtaccgaagc tcgcaggaac tcagagtgtg gagaggacga aaatgtccag gga             53


<210>   15
<211>   23
<212>   DNA
<213>   Artificial Sequence

<220>
<223>   Primer für PCR


<220>
<221>   misc_feature
<222>   (23)..(23)
<223>   Tetramethylrhodamine ist am 5'-Ende des Stranges angehängt

<400>   15
gctcatacta caatgtcact tac                                               23


<210>   16
<211>   50
<212>   DNA
<213>   Artificial Sequence

<220>
<223>   Primer für PCR


<220>
<221>   misc_feature
<222>   (50)..(50)
<223>   Tetramethylrhodamine ist am 5'-Ende des Stranges angehängt

<400>   16
aagctcgaca aaagaactca gagtgtgctc gacactacct gtattccttg                 50

<210>  17
<211>  23
<212>  DNA
<213>  Artificial Sequence

<220>
<223>  Primer für PCR



<220>
<221>  misc_feature
<222>  (23)..(23)
<223>  Tetramethylrhodamine ist am 5′-Ende des Stranges angehängt

<400>  17
gctcatacta caatgtcact tac                                              23


<210>  18
<211>  67
<212>  DNA
<213>  Artificial Sequence

<220>
<223>  PCR Produkt



<220>
<221>  misc_feature
<222>  (1)..(35)
<223>  Bindungssequenz für das erste Primer-Oligonukleotid

<220>
<221>  misc_feature
<222>  (36)..(46)
<223>  Das zweite Primer-Oligonukleotid bindet an das Reverse-Complement

<220>
<221>  misc_feature
<222>  (50)..(62)
<223>  Bindungssequenz für das dritte Primer-Oligonukleotid

<400>  18
gctcatacta caatgtcact tactgtaaga gcagatccct ggacaggcaa ggaatacagg      60

taaaaaa                                                                67


<210>  19
<211>  55
<212>  DNA
<213>  Artificial Sequence

<220>
<223>  Primer für PCR



<220>
<221>  misc_feature
<222>  (32)..(43)
<223>  2´-O-Me-Modifikationen

<220>
<221>  misc_feature
<222>  (43)..(44)
<223>  Nukleotid 43 ist mit Nukleotid 44 über einen C3-Linker verbunden
       und dient zur Terminierung der Synthese des zweiten
       Primer-Verlängerungsproduktes

<220>
<221>  modified_base
<222>  (43)..(43)
<223>  Uracil instead of thymidine

<220>
<221>  misc_feature
<222>  (44)..(55)
<223>  Segment 44 bis 55 dient als Primer

<220>
<221>  modified_base
<222>  (45)..(45)
<223>  Uracil instead of thymidine

<220>
<221>  modified_base
<222>  (48)..(48)
<223>  Uracil instead of thymidine

<220>
<221>  modified_base
<222>  (51)..(51)
<223>  Uracil instead of thymidine

<220>
<221>  modified_base
<222>  (52)..(52)
<223>  Uracil instead of thymidine

<400>  19
cacttacgac acaagatttt tttacctgta tctctgatgc ttctacctgt attcc          55


<210>  20
<211>  56
<212>  DNA
<213>  Artificial Sequence

<220>
<223>  Primer für PCR


<220>
<221>  misc_feature
<222>  (56)..(56)
<223>  Tetramethylrhodamine ist am 5'-Ende des Stranges angehängt

<400>  20
gtaccgaagc tcgcaggaac tcagagtgtg gagaggacga aaactgtcca gggatc          56


<210>  21
<211>  51
<212>  DNA
<213>  Artificial Sequence

<220>
<223>  Primer für PCR



<220>
<221>  misc_feature
<222>  (51)..(51)
<223>  Tetramethylrhodamine ist am 5'-Ende des Stranges angehängt

<400>  21
gtaccgaagc tcgcaggaac tcagagtgtg gagaggacga aaaccaggga t                51


<210>  22
<211>  53
<212>  DNA
<213>  Artificial Sequence

<220>
<223>  Primer für PCR



<220>
<221>  misc_feature
<222>  (53)..(53)
<223>  Tetramethylrhodamine ist am 5'-Ende des Stranges angehängt

<400>  22
gtaccgaagc tcgcaggaac tcagagtgtg gagaggacga aaatgtccag gga              53


<210>  23
<211>  51
<212>  DNA
<213>  Artificial Sequence

<220>
<223>  Primer für PCR



<220>
<221>  misc_feature
<222>  (51)..(51)
<223>  Tetramethylrhodamine ist am 5'-Ende des Stranges angehängt

<400>  23
gtaccgaagc tcgcaggaac tcagagtgtg gagaggacga aaactgtcca g                51

## Beschreibung

Die vorliegende Erfindung betrifft ein Verfahren zur Amplifikation von Nukleinsäuren mit verbesserter Spezifität.

### Hintergrund

Die Synthese von Nukleinsäureketten nimmt heute eine zentrale Rolle in der Biotechnologie ein. Verfahren wie PCR haben sowohl die Forschungslandschaft als auch industrielle Applikationsfelder, wie Diagnostik in der Medizin und Lebensmittelindustrie, signifikant weiterentwickelt. Die Verbindung der PCR mit anderen Technologien, wie Sequenzierung, *Real*-*Time*-Nachweis, Microarray-Technologie, Mikrofluidic-Managment etc. hat zur technologischen Entwicklung der Basistechnologie beigetragen und einige Barrieren der Grundtechnologie der PCR teilweise überwinden können. Es wurden auch weitere Amplifikations-Verfahren, wie isothermale Amplifikationstechniken (LAMP, HDA, RPA, TMA, SDA etc.) entwickelt. Deren Einsatz wurde besonders für Bereich von POCT (*point-of-care-testing*) vorgesehen.

Eine der Eigenschaften von verbreiteten Amplifikationsverfahren, wie der PCR, besteht darin, dass während des Amplifikationsvorgangs der Nukleinsäure keine Kontrolle der amplifizierten Sequenzabschnitte zwischen beiden Primern stattfindet. Im Wesentlichen steht die Primer-Bindung im Fokus von Optimierungen von PCR-Amplifikationsreaktionen. Zu Beginn der PCR-Amplifikation und in ihrem Verlauf kommt es kontinuierlich zu einer mehr oder weniger spezifischen Primer-Bindung und Initiierung der Synthese von Hauptprodukten und Nebenprodukten. Die Nebenprodukte können beispielsweise als Folge eines unspezifischen Primer-Verlängerungsereignisses in einem Synthesezyklus generiert werden. Bei ggf. erfolgenden Rücksynthesereaktionen wird der unspezifisch verlängerte Primer als Matrize abgelesen, was in der Regel zu Ausbildung einer vollständigen PrimerBindungsstelle führt. Somit erfolgt eine Übertragung einer fehlerhaften Sequenzinformation von einem Synthese-Zyklus auf den nächsten Synthese-Zyklus, was in Summe von Synthese-Zyklen nicht nur zur initialen Entstehung, sondern vor allem zur exponentiellen Vermehrung von Nebenprodukten führt.

Solche Nebenreaktionen können unter Umständen zur initialen Entstehung und exponentiellen Vermehrung von Fragmenten führen, welche die Hauptreaktion (Amplifikation einer Zielsequenz) stören bzw. zu Interferenzen in nachfolgenden Schritten der Analyse führen. Solche Nebenprodukte umfassen typischerweise Primersequenzen, deren Amplifikation parallel zu der Hauptreaktion stattfinden kann. Anstatt der gewünschten Zielsequenz umfassen solche Nebenprodukte allerdings eine andere Nukleinsäuresequenz.

Vorrangig wird die Spezifität der PCR Amplifikation durch Optimierung der Primer-Bindung an Zielsequenzen erreicht. Dabei können beispielsweise zusätzliche Oligonukleotide verwendet werden, welche an den Primer teilweise binden können und somit bei Primer-Bindung an andere Nukleinsäureketten kompetitiv mitwirken. Die Wirkung solcher Oligonukleotide ist auf die Interaktion mit Primer-Sequenzen beschränkt.

Die Nebenreaktionen bei einer PCR nehmen mit der Anzahl von durchgeführten Synthese-Zyklen zu. Dabei müssen bei manchen Anwendungen bis zu 30 - 40 PCR-Zyklen durchgeführt werden, damit eine hinreichende Menge an PCR-Produkten akkumuliert wird. Nach der PCR-Amplifikation können andere Verfahren angeschlossen werden, z.B. Detektion, *library*-Herstellung, Klonierung, Sequenzierung, etc. Diese Verfahren können nur bis zu einem gewissen Maß die während einer PCR-Amplifikation entstandenen Nebenprodukte bzw. Sequenzabweichungen tolerieren. Im Allgemeinen wird die Spezifität solcher Verfahren ebenfalls von der Problematik der Nebenreaktionen während einer PCR beeinflusst.

Im Allgemeinen kann eine Verbesserung der Spezifität der Synthese eines Amplifikationsverfahrens mit reduzierter Entstehung und Co-Amplifikation von Nebenprodukten, welche sich von Zielsequenz unterscheiden, beispielsweise zu einer Verbesserung von diagnostischen Verfahren beitragen.

Es ist daher eine Aufgabe der vorliegenden Erfindung, ein Verfahren mit verbesserter Spezifität der Synthese von Zielnukleinsäureketten in einer exponentiellen Amplifikation bereitzustellen.

Diese Aufgaben werden durch ein Verfahren mit den Merkmalen des Anspruchs 1 und ein Kit mit den Merkmalen des Anspruchs 14 gelöst. Weitere vorteilhafte Ausgestaltungen der Erfindung sind in den abhängigen Ansprüchen und der folgenden Beschreibung aufgeführt.

### Beschreibung der Erfindung

Gemäß der vorliegenden Erfindung wird ein Verfahren zur Amplifikation von Nukleinsäuresequenzen zur Verfügung gestellt, wobei
a) eine Probe, die ein erstes Nukleinsäurepolymer umfassend eine erste Zielsequenz M [und die zu M (revers) komplementäre Sequenz M'], wobei M in 5'-3'-Orientierung in unmittelbarer Folge die Sequenzabschnitte MPL, MS und MPR umfasst, in einem ersten Amplifikationsschritt mit folgenden Komponenten in Kontakt gebracht wird;
b) eine erste matrizenabhängige Nukleinsäurepolymerase, insbesondere eine DNA-Polymerase, sowie Substrate der matrizenabhängigen Nukleinsäurepolymerase (insbesondere Ribonukleosid-triphosphate oder Desoxyribonukleosid-triphosphate) und geeignete Kofaktoren;
c) ein erster linker Oligonukleotidprimer PL1, welcher (im Wesentlichen) identisch mit MPL ist;
d) ein erster rechter Oligonukleotidprimer PR1, der in 5'-3'-Orientierung in unmittelbarer Folge die Sequenzabschnitte PCR und PMR umfasst, wobei PMR die zu MPR komplementäre [hybridisierende] Sequenz aufweist [im Wesentlichen sequenzspezifisch binden kann] und der Sequenzabschnitt PCR nicht an M [oder eine in Bezug auf die Sequenz M in 3' unmittelbar an MPR anschließende Sequenz] binden kann, und
   wobei PR1 (insbesondere im Abschnitt PCR) modifizierte Nukleotidbausteine umfasst, so dass PCR nicht als Matrize für die Aktivität der ersten matrizenabhängigen Nukleinsäurepolymerase dienen kann; und
e) ein Controller-Oligonukleotid CR, das in 5'-3'-Orientierung die Sequenzabschnitte CSR, CPR und CCR in unmittelbarer Folge umfasst, wobei CSR identisch zu einem Abschnitt von MS ist, welcher in 5' von MPR liegt [und bei Initiation der Polymerase von PR zuerst abgelesen wird, CSR also mindenstens an einen Teil des Polymerisationsprodukts bzw. Verlängerungsprodukts des Primers PR1 binden kann], CPR komplementär zu PMR (und identisch zu MPR) ist und CCR komplementär zu PCR ist bzw. CCR eine zu PCT komplementäre Sequenz aufweist und an PCR binden kann,
   und wobei CR in CSR und/oder CPR modifizierte Nukleotidbausteine umfasst, so dass CSR nicht als Matrize für die Aktivität der matrizenabhängigen Nukleinsäurepolymerase dienen kann; und
   wobei die Probe in einem zweiten Amplifikationsschritt mit folgenden Komponenten in Kontakt gebracht wird:
   a) ein zweiter linker Oligonukleotidprimer PL2, welcher zu einem ersten Sekundärprimerbindungsbereich MPL2 identisch ist, und
   b) ein zweiter rechter Oligonukleotidprimer PR2, welcher zu einem Bereich MPR2 komplementär ist,
      wobei MPL2 und MPR2 von M umfasst werden und das 3'-Ende von MPL2 [mindestens 5, 10 oder 20 Positionen] in 5'(-Richtung) des 5'-Endes von MPR2 angeordnet ist, (insbesondere MPL2 mit MPL und /oder MPR2 mit MPR identisch ist)

Insbesondere ist eine Nukleinsäuresequenz am 3'Ende von CSR mit einer Länge von mindestens 10 Nukleotiden, insbesondere die gesamte Nukleoinsäuresequenz von CSR, identisch zu einem Abschnitt von MS, insbesondere einem Abschnitt am 3'Ende von MS, ist, welcher in 5' von MPR liegt [und bei Initiation der Polymerase von PR zuerst abgelesen wird. In einigen Ausführungsformen des erfindungsgemäßen Verfahrens ist vorgesehen, dass ein erstes Primer-Verlängerungsprodukt PR1' (als Ergebnis einer polymerasenabhängigen Synthese unter Verwendung von PR1 als Primer spezifisch gebunden and die entsprechenden Matrizen (M oder PL1')), erhalten wird, welches in 5'-3'-Orientierung neben den Sequenzbereichen PCR und PMR einen synthetisierten Bereich PSR umfasst, der im Wesentlichen komplementär zur Zielsequenz M in dem in 5' an MPR angrenzenden Bereich ist, und insbesondere eine zu MPL komplementäre Sequenz umfasst, und ein zweites Primer-Verängerungsprodukt PL1' (als Ergebnis einer polymerasenabhängigen Synthese unter Verwendung von PL1 als Primer spezifisch gebunden an die entsprechende Matrizen (M' oder PR1')), erhalten wird, welches neben dem Sequenzbereich MPL einen synthetisierten Bereich PSL umfasst, der im Wesentlichen identisch zur Zielsequenz M im an MPL in 3' angrenzenden Bereich ist und eine zu MPR im Wesentlichen identische Sequenz umfasst bzw. die Zielsequenz M im an MPL in 3'angrenzenden Bereich umfasst und Sequenzabschnitt MPR umfasst, und
- entweder die Reaktionsbedingungen des ersten Amplifikationsschrittes so gewählt sind, und/oder
- die Längen und Schmelztemperaturen von PCR und ggf. MS so gewählt sind,
dass PR1' mit M oder mit PL1'einen Doppelstrang bilden kann, [PL1' mit M' oder PR1'einen Doppelstrang bilden kann,] [PR1'mit PL1'einen Doppelstrang bilden kann], und PR1' mit C einen Doppelstrang bilden kann und die Bildung des Doppelstranges aus PR1' und C gegenüber der Bildung des Doppelstrangs aus PR1' mit M [mindestens im Bereich von MPR] bevorzugt ist bzw. zu Trennung von PR1' von M oder zu Trennung von PR1'von PL1' führen kann .

In einigen Ausführungsformen des erfindungsgemäßen Verfahrens ist vorgesehen, dass insbesondere ein drittes Primer-Verlängerungsprodukt PL2'erhalten wird, welches einen synthetisierten Bereich aufweist, der zum Bereich MPR2 identisch ist, und ein viertes Primer-Verlängerungsprodukt PR2'erhalten wird, der einen synthetisierten Bereich aufweist, der zum Bereich MPL2 komplementär ist.

Insbesondere werden die im ersten Amplifikationsschritt erhaltenen PL1' und PR1' als Matrizen für exponentielle Amplifikation unter Verwendung der Primer PL2 und PR2 und der ersten matrizenabhängigen Nukleinsäurepolymerase aus Schritt b) oder einer anderen matzizenabhängigen Nukleinsäurepolymerase im zweiten Amplifikationsschritt verwendet. Hierbei entstehen insbesondere die Primerverlängerungsprodukte PL2' und PR2'.

Insbesondere sind die Reaktionsbedinungen des zweiten Amplifikationsschrittes derart gewählt [bevorzugt verläuft der zweite Amplifikationsschritt als Polymerasen-Ketten-Reaktion], dass eine exponentielle Amplifikation der Primerverlängerungsprodukte PL2' und PR2' unter Verwendung von PL2 und PR2 als Primer, sowie einer geeigneten matrizenabhängigen Nukleinsäurepolymerase und entsprechenden Kofaktoren erfolgen kann, wobeiPL2 mindestens mit seinem 3'-Segment an das im ersten Amplifikationsschritt gebildete PR1' komplementär binden kann und von Polymerase im zweiten Amplifikationsschritt verlägert werden kann, wobei ein Verlängerungsprodukt PL2' erhalten wird, und PR2 mindestens mit seinem 3'-Segment an das im ersten Amplifikationsschritt gebildete PL1' komplementär binden kann und von Polymerase im zweiten Amplifikationsschritt verlägert werden kann, wobei ein Verlängerungsprodukt PR2' erhalten wird, wobei weiterhin PR2 an PL2' komplementär binden kann und von Polymerase im zweiten Amplifikationsschritt verlägert werden kann, wobei ein Verlängerungsprodukt PR2' erhalten wird, wobei weiterhin PL2 an PR2' komplementär binden kann und von Polymerase im zweiten Amplifikationsschritt verlägert werden kann, wobei ein Verlängerungsprodukt PL2' erhalten wird.

Vorzugweise wird die zweite Amplifikationsreaktion als PCR durchgeführt.

In einigen Ausführungsformen der Erfindung ist vorgesehen, dass
- entweder die Reaktionsbedingungen des ersten Amplifikationsschrittes so gewählt sind, und/oder
- die Längen und Schmelztemperaturen von PCR und ggf. MS und/oder die Positionen von MPL und MPR so gewählt sind,
dass sich in Abwesenheit des Controller-Oligonukleotides CR das erste Primer-Verlängerungsprodukt PR1' nicht vom zweiten Primer-Verlängerungsprodukt PL1' trennt.

In einigen Ausführungsformen des erfindungsgemäßen Verfahrens ist vorgesehen, dass PL2 und/oder PR2 [unmittelbar] in 5' von dem zu MPL2 identischen bzw. dem zu MPR2 komplementären Sequenzabschnitt einen Sequenzabschnitt PCL2 bzw. PCR2 aufweisen.

Der Begriff "im Wesentlichen indentisch" bedeutet im Sinne der Erfindung insbesondere, dass zwei Nukleinsäuren nicht mehr als 5, 4, 3, 2, oder 1 Mismatches zueinander aufweisen.

Der Begriff "im Wesentlichen komplementär" bedeutet im Sinne der Erfindung insbesondere, dass die zueinander komplementären Bereiche von Nukleinsäuren nicht mehr als 5, 4, 3, 2, oder 1 Mismatches aufweisen.

In einer weiteren Ausführungsform des erfindungsgemäßen Verfahrens ist vorgesehen, dass eine zweite matrizenabhängige Nukleinsäurepolymerase, insbesondere eine DNA-Polymerase, sowie ggf. Substrate der matrizenabhängigen Nukleinsäurepolymerase (insbesondere Ribonukleosid-triphosphate oder Desoxyribonukleosid-triphosphate) und geeignete Kofaktoren), in dem zweiten Amplifikationsschritt mit der Probe in Kontakt gebracht wird.

In einigen Ausführungsformen des erfindungsgemäßen Verfahrens ist vorgesehen, dass die modifizierten Nukleotidbausteine 2'-O-Alkylribonukleosidbausteine, insbesondere 2'-O-Methylribonukleosidbausteine, umfassen.

In einer Ausführungsform des erfindungsgemäßen Verfahrens ist vorgesehen, dass die Schritte der ersten Amplifikation so oft wiederholt werden, bis das erste Amplifikations-Fragment umfassend das erste Primerverlängerungsprodukt und das zweite Primerverlängerungsprodukt in einer Kopienanzahl im Bereich von 10 bis 100.000.000.0000 vorliegt, insbesondere von 100 bis 1000.000.000, insbesondere von 1000 bis 100.000.000.

In einer weiteren Ausführungsform des erfindungsgemäßen Verfahrens ist vorgesehen, dass die zweite Polymerase eine thermostabile Polymerase ist, insbesondere eine thermostabile DNA-Polymerase.

In einer weiteren Ausführungsform des erfindungsgemäßen Verfahrens ist vorgesehen, dass erste Polymerase thermisch inaktivierbar ist, insbesondere dass die erste matrizenabhängige Nukleinsäurepolymerase eine mesophile Polymerase ist.

In einer weiteren Ausführungsform des erfindungsgemäßen Verfahrens ist vorgesehen, dass die erste Amplifikation und die zweite Amplifikation im selben Reaktionsansatz durchgeführt werden.

In einer weiteren Ausführungsform des erfindungsgemäßen Verfahrens ist vorgesehen, dass die zweite Polymerase, das dritte Primer-Oligonukleotid und/oder das vierte Primer-Oligonukleotid aktivierbar sind, und nsbesondere vor oder während des zweiten Amplifikationsschritt aktiviert wird oder werden, und/oder das Controller-Oligonukleotid deaktivierbar ist, und insbesondere vor oder während des zweiten Amplifikationsschritt deaktiviert wird.

In einer Ausführungsform des erfindungsgemäßen Verfahrens ist vorgesehen, dass die erste Polymerase vor Durchführung der zweiten Amplifikation inaktiviert wird. Dies kann beispielsweise durch thermale Denaturierung erreicht werden, wenn es sich bei der ersten Polymerase um eine nicht thermostabile Polymerase handelt.

In einer weiteren Ausführungsform des erfindungsgemäßen Verfahrens ist vorgesehen, dass die erste Amplifikation im Wesentlichen isothermal durchgeführt wird.

In einer weiteren Ausführungsform des erfindungsgemäßen Verfahrens ist vorgesehen, dass MS eine Länge von 20 Nukleotiden bis 200 Nukleotiden hat. In einigen Ausführungsformen des erfindungsgemäßen Verfahrens ist vorgesehen, dass PR1 eine Länge im Bereich von 15 Nukleotiden bis 100 Nukleotiden hat. In einigen Ausführungsformen des erfindungsgemäßen Verfahrens ist vorgesehen, dass PC eine Länge im Bereich von 5 Nukleotiden bis 85 Nukleotiden hat, insbesondere dass PCR zwischen 50% und 300% der Länge des Sequenzabschnitts PMR hat. In einigen Ausführungsformen des erfindungsgemäßen Verfahrens ist vorgesehen, dass C eine Länge im Bereich von 20 Nukleotiden bis 100 Nukleotiden hat.

In einer weiteren Ausführungsform des erfindungsgemäßen Verfahrens ist vorgesehen, dass die Komponenten des zweiten Amplifikationsschrittes nach Durchführung des ersten Amplifikationschritts mit der Probe in Kontakt gebracht werden.

In einer weiteren Ausführungsform des erfindungsgemäßen Verfahrens ist vorgesehen, dass CR in CSR und/oder CPR einen Sequenzbereich umfasst, der sequenzspezifisch an den zweiten rechten Oligonukleotidprimer PR2 (komplementär) binden kann, wobei der Sequenzbereich insbesondere modifizierte Nukleotidanaloga umfasst, welche eine Verwendung von CR als Matrize verhindern.

Gemäß einem weiteren Aspekt der Erfindung wird ein Kit zur Durchführung des erfindungsgemäßen gemäß dem ersten Aspekt oder einem der dazugehörigen Ausführungsformen oder Alternativen zur Verfügung gestellt. Das erfindungsgemäße Kit umfasst:
- ein erster rechter Oligonukleotidprimer PR1, der in 5'-3'-Orientierung in unmittelbarer Folge die Sequenzabschnitte PCR und PMR umfasst, wobei PMR an eine Primerbindungsstelle MPR einer genomischen Sequenz M eines eukaryoten Organismus oder eines pathogenen Bakteriums, insbesondere eines Säugetiers, ganz inbesondere an eine menschliche Zielsequenz, binden kann, wobei M in 5'-3'-Orientierung in unmittelbarer Folge die Sequenzabschnitte MPL, MS und MPR umfasst, und der Sequenzabschnitt PCR nicht an eine direkt in 3' von MPR liegende Sequenz binden kann, und wobei
   PR1 (insbesondere im Abschnitt PCR) modifizierte Nukleotidbausteine umfasst, so dass PCR nicht als Matrize für die Aktivität der ersten matrizenabhängigen Nukleinsäurepolymerase dienen kann; und
- ein erster linker Oligonukleotidprimer PL1, welcher (im Wesentlichen) identisch mit MPL ist;
- ein Controller-Oligonukleotid CR, das in 5'-3'-Orientierung die Sequenzabschnitte CSR, CPR und CCR in unmittelbarer Folge umfasst, wobei CSR identisch zu einem Abschnitt von MS ist, welcher in 5' von MPR liegt [und bei Initiation der Polymerase von PR zuerst abgelesen wird, CSR also an das Polymerisationsprodukt des Primers PR1 binden kann], CPR komplementär zu PMR (und identisch zu MPR) ist und CCR komplementär zu PCR ist bzw. CCR eine zu PCR komplementäre Sequenz umfasst, und
- wobei CR in CSR modifizierte Nukleotidbausteine umfasst, so dass CSR nicht als Matrize für die Aktivität der matrizenabhängigen Nukleinsäurepolymerase dienen kann;
- ein zweiter linker Oligonukleotidprimer PL2, welcher zu einem ersten Sekundärprimerbindungsbereich MPL2 identisch ist, wobei insbesondere zumindest das 3'-Segment von MPL2 innerhalb von MPL oder MS liegt, und
- ein zweiter rechter Oligonukleotidprimer PR2, welcher zu einem Bereich MPR2 komplementär ist, wobei insbesondere zumindest das 3'-Segment von MPR2 innerhalb von MS oder MPR liegt,
- wobei MPL2 und MPR2 von M umfasst werden und das 3'-Ende von MPL2 [mindestens 20 Positionen] in 5' des 5'-Endes von MPR2 angeordnet ist, (insbesondere MPL2 mit MPL und /oder MPR2 mit MPR identisch ist).

In einer Ausführungsform des erfindungsgemäßen Kits umfasst das Kit weiterhin mindestens eine Polymerase.

In einer Ausführungsform des erfindungsgemäßen Kits umfasst das Kit eine erste Polymerase, die zur Verlängerung des ersten rechten und linken Primer-Oligonukleotids vorgesehen ist, und eine zweite Polymerase, die zur Verlängerung des zweiten rechten und linken Primer-Oligonukleotids vorgesehen ist.

Weitere Einzelheiten und Vorteile der Erfindung sollen in nicht beschränkender Weise durch die nachfolgende Figuren und einer detaillierte Beschreibung ausgewählter Ausführungsformen erläutert werden.

### Beschreibung der Figuren

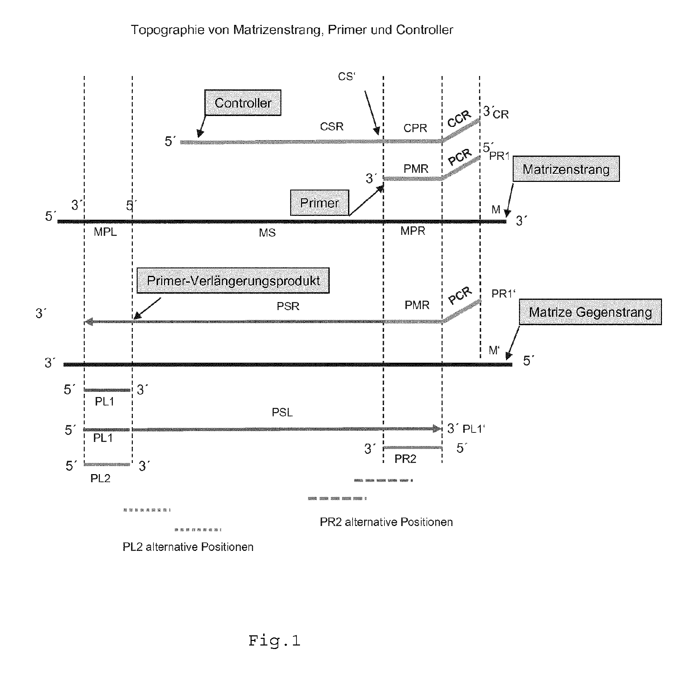

- Fig. 1: zeigt schematisch die Topographie von Matrizenstrang, Primer und Controller;

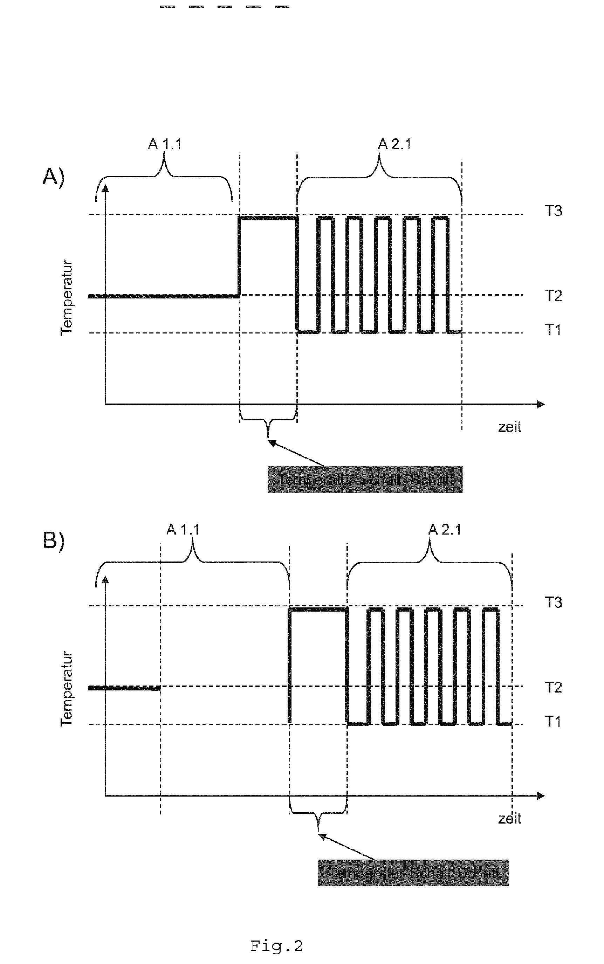

- Fig. 2: zeigt ein Temperaturprofil einer Ausführungsform des erfindungsgemäßen Verfahrens;

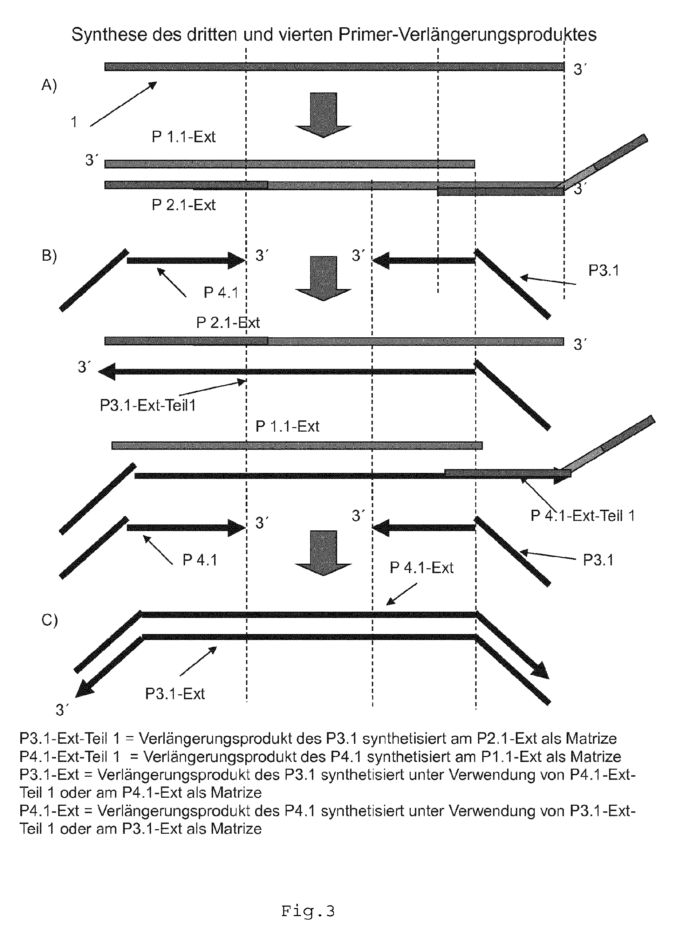

- Fig. 3: zeigt schematisch die Synthese des partiellen dritten Primer-Verlängerungsprodukts;
- Fig. 4 bis 14: zeigen die Ergebnisse von Beispielversuchen.

### Beispiele

### Material und Methoden:

Amplifikationsreaktion Lösung 1: Kalium Glutamat, 50 mmol/l, pH 8,0; Magnesium Acetat, 10 mmol/l; dNTP (dATP, dCTP, dTTP, dGTP), je 200 µmol/l; Polymerase (Bst 2.0 WarmStart, 120.000 U/ml NEB), 12 Units / 10 µl; Triton X-100, 0,1% (v/v); EDTA, 0,1 mmol/l; TPAC (Tetrapropylammonium Chloride), 50 mmol/l, pH 8,0; EvaGreen Farbstoff (Farbstoff wurde entsprechend Anleitung des Herstellers in Verdünnung 1:50 eingesetzt).

Amplifikationsreaktion Lösung 2: 1x Isothermal Puffer (New England Biolabs); dNTP (dATP, dCTP, dUTP, dGTP), je 200 µmol/l; EvaGreen Farbstoff (Farbstoff wurde entsprechend Anleitung des Herstellers in Verdünnung 1:50 eingesetzt)

Amplifikationsreaktion Lösung 4: 1x Isothermal Puffer (New England Biolabs); dNTP (dATP, dCTP, dTTP, dGTP), je 200 µmol/l.

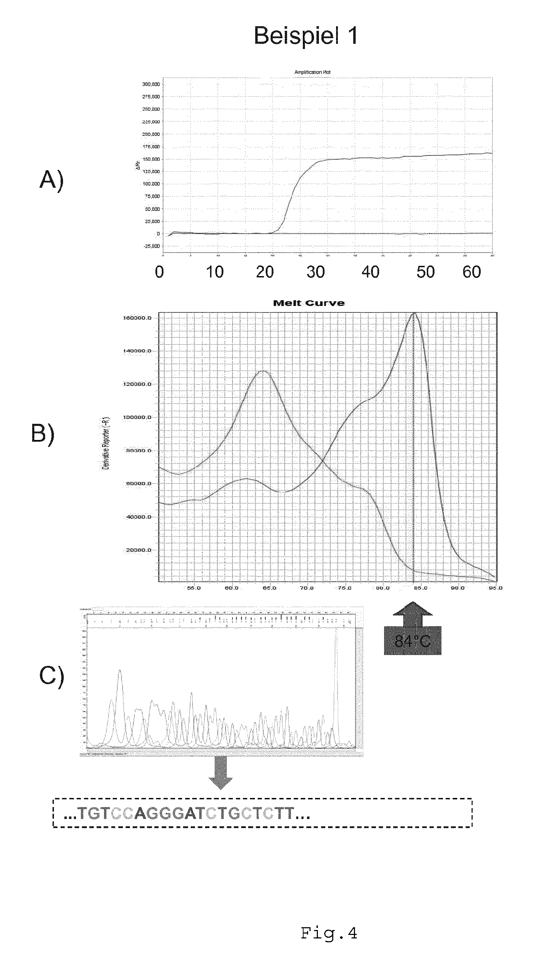

### Beispiel 1 (Fig. 4)

### Verwendung humaner genomischer DNA als Quelle von Zielsequenz

In diesem Beispiel wird die Verwendung humaner genomischer DNA (hgDNA) als Quelle einer Zielsequenz (SEQ ID NO 1) gezeigt. Als Zielsequenz wurde ein Sequenzsegment des Faktor-V-Leiden Genes (Homo sapiens coagulation factor V (F5), mRNA, hier als FVL-Gen bezeichnet) gewählt.

Der erste Primer (P1F5-200-AE2053, SEQ ID NO 2) der zweite Primer (P2G3-5270-7063, SEQ ID NO 3) sowie Controller-Oligonukletid (AD-F5-1001-503, SEQ ID NO 4) wurden für FVL-Mutation Variante des Genes designet und synthetisiert.

Der erste Primer umfasst in seinem ersten Bereich eine Sequenz, welche spezifisch an die Sequenz des Faktor-V-Leiden Genes innerhalb der genomischen DNA binden kann, so dass eine Synthese durch eine Polymerase gestartet werden kann. Der zweite Bereich des ersten Primers umfasst eine Sequenz, welche nicht an die Sequenz des FVL-Genes spezifisch hybridisiert. Weiterhin umfasst der erste Primer ein weiteres Sequenzsegment, welches an das 5'-Ende des zweiten Bereichs anknüpft. Dieses Segment nimmt an der spezifischen Amplifikation des Faktor-5-Leiden Segments nicht teil. Die Funktion dieses Segments wird vor allem in der Verzögerung von Nebenreaktionen gesehen.

Der zweite Primer umfasst ein Segment in seinem 3'-Segment, welches spezifisch an die genomiche DNA binden kann, so dass eine Synthese durch eine Polymerase gestartet werden kann. Das 5'-Segment des zweiten Primers umfasst eine Sequenz, welche nicht an die Sequenz des FVL-Genes spezifisch hybridisiert. Während der Rücksynthese kann dieses Sequenzsegment kopiert werden. Der zweite Primer umfasst ein weiteres Sequenzsegment, welches weder mit dem Controller-Oligonukleotid, noch mit dem ersten Primer, noch mit dem zweiten Primer spezifisch hybridisieren kann. Dieses Segment wurde an das 5'-Ende des zweiten Primer lokalisiert und ist vom 5'-Ende des Primers durch eine HEG-Linker getrennt.

Dieses Segment nimmt nicht an der spezifischen Amplifikation teil. Die Funktion dieses Segments wird vor allem in der Verzögerung von Nebenreaktionen gesehen.

Das Controller-Oligonukleotid wurde dermaßen konstruiert, dass eine Perfekt-Match-Situation zur Sequenz der Faktor-V-Leiden Mutation des FVL-Gens resultiert. Das Controller-Oligonukleotid umfasst einen ersten, zweiten und dritten Bereich.

Als genomische DNA wurde der WHO-Standard für FVL-Mutation verwendet. Vor der Verwendung in der Reaktion wurde die DNA durch Erhitzung denaturiert (5 min bei 95°C) und damit aus dem doppelsträngigen Zustand in den einzelsträngigen Zustand überführt. Anhang dieser einzelsträngiger hgDNA wurde zunächst eine Start-Nukleinsäurekette durch eine Primer-Verlängerng erstellt. Anschließend erfolgte eine exponentielle Amplifikation ausgehend von dieser Start-Nukleinsäurekette. Der Nachweis der Spezifität der Amplifikation erfolgte mittels Schmelzkurven-Analyse und Sanger-Sequenzierung mit einem Sequenzierungprimer.

Die Start-Nukleinsäurekette wurde wie folgt erstellt: Etwa 50000 haploider genom Äquivalente (HGE), 150 ng hgDNA, wurden in Kontakt mit dem zweiten Primer (0,5 µmol/l) und Bst-2.0 Warm Start Polymerase (etwa 1 unit), sowie dNTPs (ca. 250 µmol/l) unter Hybridierungsbedingungen (Amplifikationslösung 1, Temperatur von etwa 60°C) in 50 µl Reaktionsvolumen gebracht und ca. 10 min inkubiert. Während dieser Phase erfolgt eine Verlängerung des zweiten Primers, wobei die genomische DNA als Matrize dient. Es resultiert ein Primer-Verlängerungsprodukt, welches als Start-Nukleinsäure dienen kann. Nach Abschluss dieser Reaktion wurde das Reaktionsgemisch erhitzt auf 95°C für ca. 10 min, um diese Start-Nukleinsäurekette von der Matrize zu trennen. Dieses Reaktionsgemisch wurde eingefroren und als Quelle der Start-Nukleinsäurekette nach Bedarf verwendet.

Die spezifische Amplifikation der Zielsequenz des FVL-Gens erfolgte unter Verwendung von 5 µl des Reaktionsgemisches mit der Start-Nukleinsäurekette (entspricht ca. 5000 HGE). Die anderen Reaktionskomponenten (erster Primer, zweiter Primer, Controller-Oligonukleotid, Eva-Green Farbstoff, Polymerase Bst.2.0 Warm Start, dNTPs) wurden dazugegeben, so dass ein Reaktionsendvolumen von ca. 10 µl resultierte. Die Endkonzentrationen der Komponenten betrugen: erster Primer: 5µmol/l, zweiter Primer: 2 µmol/l, Controller-Oligonukleotid: 1 µmol/l, Eva-Green Farbstoff (1:50), Polymerase Bst.2.0 Warm Start (ca. 8 Units), dNTPs: ca. 250 µmol/l. Im Kontroll-Ansatz wurde keine hgDNA zugegeben.

Die Reaktion wurde in einem Step-One Plus Gerät (Thermofisher Scientific) durchgeführt. Die Reaktionstemperatur wurde initial durch zyklische Änderungen (30 Zyklen) geändert zwischen 65°C (5 min, einschließlich Detektionsschritt) und 55°C (1 min) und anschließend für 1 hr bei 65°C konstant gehalten (Detektionsschritt alle 2 min). Der Verlauf der Reaktion wurde durch Signal-Erfassung des EvaGreen Farbstoffs verfolgt. Nach Abschluss der Reaktion wurde das Reaktionsgemisch zunächst auf 95°C für 10 min gebracht und anschließend wurde eine Schmelzkurve der gebildeten Produkte gemessen.

Nach Erstellung der Start-Nukleinsäurekette erfolgte eine exponentielle Amplifikation unter Verlängerung des ersten Primers und des zweiten Primers und des Controller-Oligonukleotids.

Als Ergebnis der Amplifikation kommt es zu Akkumulierung von Amplifikationsfragmenten. Das Ergebnis der Detektion der Amplifikation ist in Fig. 4 zu sehen. Man sieht, dass ca. nach 2 hr der Reaktion eine sichtbare Zunahme der Fluoreszenz erfolgt (Fig. 4 A). Anschließende Schmelzkurven-Analyse zeigte eine spezifische Schmelzkurve mit Tm von 84°C (Fig. 4 B). Die Sequenz wurde mittels eines Sequenzierungsprimers (SEQP1F5-35-X03, SEQ ID NO:5) überprüft (Fig.45C).

Zur Sequenzüberprüfung wurde das Reaktionsgemisch (nach Messung der Schmelzkurve) mit Wasser verdünnt (von ca. 1:10 bis ca. 1:100) und die jeweils erhaltenen Aliquoten mit einem Sequenzierungsprimer (zugegeben in Konzentration von 2 µmol/l) gemischt. Dieses Gemisch wurde durch einen kommerziellen Sequenzierungs-Anbieter versendet (GATC-Biotec) und mittels Sanger-Sequenzierung als Auftrags-Sequenzierung sequenziert. Die erhaltenen Elektropherogramme wurden auf Übereinstimmung mit der FVL-Sequenz Gens geprüft. Als Ergebnis der Reaktion wurde die Sequenz des FVL-Gens identifiziert.

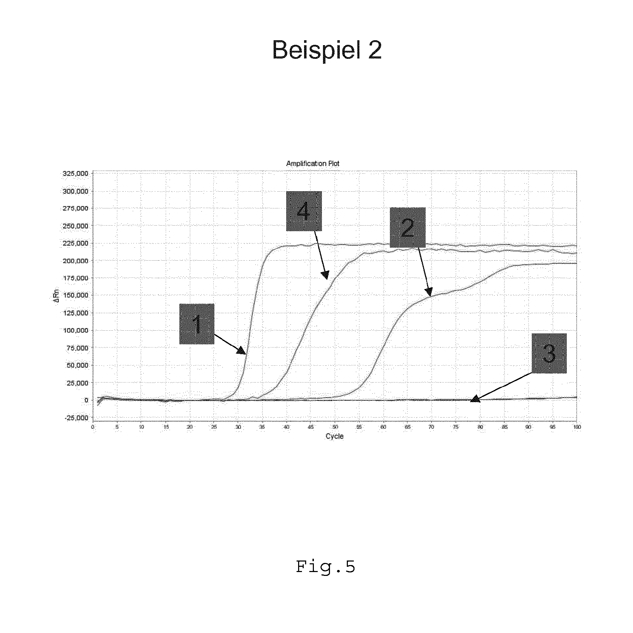

### Beispiel 2 (Fig. 5):

*Selektive Amplifikations-Reaktion von Sequenz-Varianten einer Zielsequenz.*

In diesem Beispiel wurde der Einfluss einer Sequenzänderung in der Matrize auf die Amplifikation untersucht. Bei Verlängerung des ersten Primer-Oligonukleotides wird ein komplementärer Strang gebildet, welcher eine komplementäre Sequenz zur Matrize aufweist und somit diese Abweichungen in der Sequenz umfasst. Auf diese Weise sollte überprüft werden, welche Auswirkung ein solcher Mismatch zwischen einem dabei entstehenden ersten Primer-Verlängerungsprodukt und einem Controller-Oligonukleotid auf die Amplifikation hat. Die Position des Mismatches liegt in 3'-Richtung vom ersten Primer und wird somit nicht vom Primer, sondern durch das Controller-Oligonukleotid überprüft. Die Diskriminierung zwischen einzelnen Sequenz-Varianten der Zielsequenz erfolgt somit mittels Controller-Oligonukleotides unter Verwendung eines einheitlichen ersten und zweiten Primers.

Folgende Kompenenten wurden verwendet:
- Matrize M2SF5-M001-200 (SEQ ID NO 6) mit Sequenzzusammensetzung, welche zu einem ersten Primer-Verlängerungsprodukt mit einer Perfekt-Match Übereinstimmung mit Controller-Oligonukleotid führt:
- Matrize M2SF5-WT01-200 (SEQ ID NO 7) mit Sequenzzusammensetzung, welche zu einem ersten Primer-Verlängerungsprodukt führt, das an einer einzelnen Basenposition ein Mismatch mit dem Controller-Oligonukleotid bildet.
- erstes Primer-Oligonukleotid P1F5-200-AE2053 (SEQ ID NO:2);
- zweites Primer-Oligonukleotid P2G3-5270-7063 (SEQ ID NO:8);
- Controller-Oligonukleotid AD-F5-1001-503 (SEQ ID NO:4) wurde verwendet.

Es wurden vier Ansätze vorbereitet:
Ansatz 1 enthält als Start-Nukleinsäurekette die Matrize M2SF5-M001-200 (Perfect Match Situation) in Konzentration von 300 fmol/l (entspricht ca. 2x10^6 Kopien/ Ansatz).
Ansatz 2 enthält als Start-Nukleinsäurekette die Matrize M2SF5-M001-200 (Perfect Match Situation) in Konzentration von 300 amol/l (entspricht ca. 2x10^3 Kopien/ Ansatz).
Ansatz 3 enthält keine Matrize und bildet somit eine Kontrolle.
Ansatz 4 enthält als Start-Nukleinsäurekette die Matrize M2SF5-WT01-200 (single Mismatch Situation) in 300 pmol/l Konzentration (ca. 2x 10^9 Kopien pro Ansatz).

Primer 1 wurde mit 5 µmol/l, das Controller-Oligonukleotid mit 2 µmol/l und Primer 2 mit 1 µmol/l eingesetzt. Die Reaktionen wurden in Amplifikations-Lösung 2 durchgeführt.

Um die Anwesenheit von genomischer DNA im Assay nachzustellen wurden pro Reaktion 100 ng frisch denaturierte Fisch-DNA (Lachs-DNA) zugegeben. Die thermischen Reaktionsbedingungen waren zyklisch, alternierende Temperaturänderungen, wobei jeweils auf ein 2 min Zeitintervall bei 55 °C ein 5 min Zeitintervall bei 65 °C folgte. Die Amplifikation wurde über 100 Zyklen verfolgt. Detektion erfolgte bei 65°C für EvaGreen-Fluoreszenzsignal. Die erfolgreiche Amplifikation konnte durch einen Anstieg des EvaGreen-Fluoreszenzsignals im Laufe der Zeit festgestellt werden. Die Temperatur-Änderungen und Real-Time Monitoring wurde mit StepPne Plus Real-Time PCR Gerät von Thermofisher durchgeführt.

Fig. 5 zeigt einen typischen Verlauf des EvaGreen-Signals. Auf der Y-Achse ist die Zunahme des Fluoreszenzsignal (Delta Rn) und auf der X-Achse ist die Reaktionszeit (als Zyklusnummer) aufgetragen. Pfeile markieren einzelne Reaktionsansätze. Dabei gehören die markierten Positionen zu folgenden Ansätzen:

| | |
|---|---|
| Pfeil 1: 300 fmol/l perfekt-Match Matrize | (ca. 2x10^6 Kopien/Ansatz) |
| Pfeil 2: 300 amol/l perfekt-Match Matrize | (ca. 2x10^3 Kopien/Ansatz) |
| Pfeil 3: keine Matrize | |
| Pfeil 4: 300 pmol/l Mismatch Matrize | (ca. 2x 10^9 Kopien pro Ansatz). |

Ein Anstieg des Fluoreszenz-Signals kann sowohl bei perfekt-Match als auch bei Mismatch-Variante der Matrize beobachtet werden, wobei das Signal der Mismatch-Variante (4) trotz 100fachen Überschußes später erscheint. Es ist erkennbar, dass die Mismatch-Amplifikationssignale gegenüber dem Perfekt-Match Amplifikationssignal deutlich verzögert sind. Beim Single Mismatch wird eine Verzögerung von ca. 15 Zyklen beobachtet. Die zeitliche Verzögerung (=Zyklusanzahl) ist ein unmittelbares Maß für die Diskriminierung in der Amplifikation. Eine weitere Quantifizierung der Diskriminierung in der Amplifikation kann über den Vergleich mit einer Matrizen-Konzentrationsreihe unter Perfekt-Match Amplifikation erzielt werden.

Bei Verwendung einer Perfect-Match Matrize kommt es zur Synthese eines komplementären Stranges eines Primer-Verlängerungsproduktes. Dieses Verlängerungsprodukt ist sowohl zur Perfect-Match-Matrize komplementär als auch zum verwendeten Controller-Oligonukleotid.

Im Gegensatz dazu, unter Verwendung einer Mismatch-Sequenz kommt es bei der Synthese des ersten Primer-Verlängerungsproduktes zur Generierung eines komplementären Stranges des Verlängerungsproduktes, welches zwar eine vollständige Komplementarität zur Mismatch-Matrize aufweist, aber dadurch von der Komplementarität mit dem dritten Bereich des Controller-Oligonukleotid abweicht. Diese Abweichung findet im 5'-ständigen Segment des Verlängerungsproduktes statt, welches mit dem Controller-Oligonukleotid reagieren sollte, damit der Strangverdrängungsvorgang voranschreiten kann. Wie im vorliegenden Beispiel gezeigt wird, stört das Mismatch eine Strangverdrängung durch das Controller-Oligonukleotid.

Die Kontrollreaktionen mit einer Perfect Match Matrize (Pfeile 1 und 2) zeigten eine Konzentrationsabhängigkeit der Amplifikation. Mit sinkender Konzentration brauchte die Reaktion längere Zeit, um eine ausreichende Menge der zu amplifizierenden Nukleinsäure zu synthetisieren, damit das Signal über das Niveau der Baseline ansteigt.

Dieses Ergebnis veranschaulicht die Bedeutung der Basenzusammensetzung im Controller-Oligonukleotid: Abweichungen von der Komplementarität zwischen Controller-Oligonukleotid und dem Primer-Verlängerungsprodukt können zu Verlangsamung oder sogar zu Unterbrechung der Amplifikation führen.

In diesem Beispiel wurde gezeigt, dass obwohl Sequenz-Enden von Perfect-Match-Matrize und Mismatch-Matrize komplett übereinstimmten und somit das Potenzial zur Bindung von beiden Primer-Oligonukleotide gleich war, sind beide Reaktionen vollkommen unterschiedlich gelaufen: bei einer vollständigen Komplementarität zwischen dem Controller-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall durch ein Mismatch) führte zur Unterdrückung der Amplifikation.

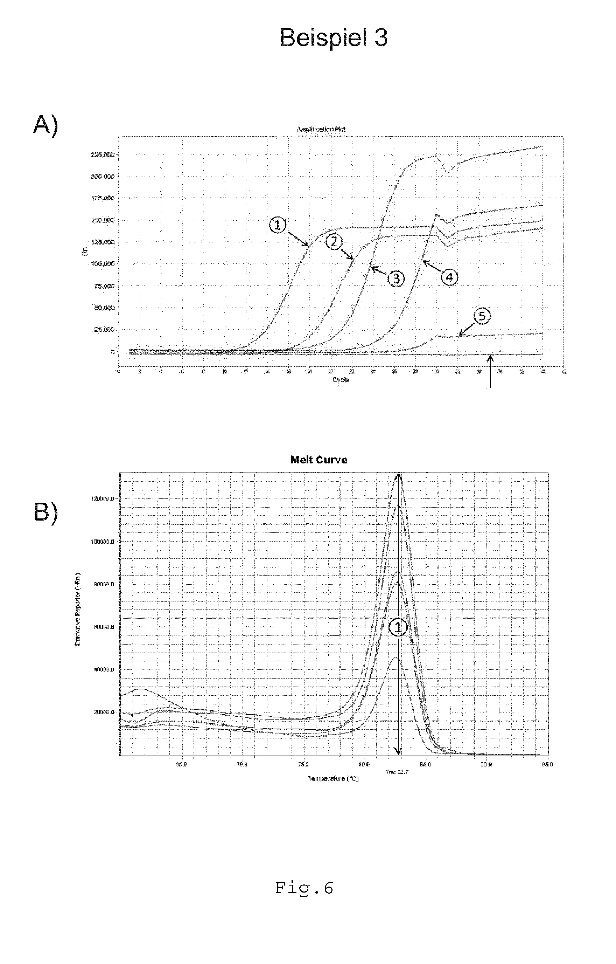

### Beispiel 3 (Fig. 6 - 10):

### Verwendung von zwei Amplifikationen einer ersten Amplifikation und nachgeschaltet einer zweiten Amplifikation

In diesem Ausführungsbeispiel wird demonstriert, dass Amplifikations-Produkte der ersten Amplifikation (erste Amplifikation) durch eine nachgeschaltete, klassische PCR (zweite Amplifikation) als Start-Nukleinsäureketten verwendet werden können. Somit wurden insgesamt zwei getrennte Amplifikations-Reaktionen durchgeführt: zunächst eine erste Amplifikation, danach eine zweite Amplifikation.

### Erste Amplifikation

Zunächst wurde eine erste Amplifikation durchgeführt unter Verwendung einer einzelsträngigen Nukleinsäurekette (Start-Nukleinsäurekette 1.1), welche eine Zielsequenz umfasste, und eines ersten Amplifikations-Systems. Die verwendeten Reaktionsbedingungen (Temperatur von 55°C und 65°C) lassen keine spontane Trennung des während der Reaktion gebildeten Doppelstranges, umfassend das erste Primer-Verlängerungs-Produkt und das zweite Primer-Verlängerungsprodukt, in Abwesenheit eines Controller-Oligonukleotides zu.

Das erste Amplifikations-System umfasste folgende Komponenten:
- Ein erstes Primer-Oligonukleotid (P1F5-200-AE205, SEQ ID NO 9),
- ein zweites Primer-Oligonukleotid (P2G3-5270-7063, SEQ ID NO 8),
- ein Controller-Oligonukleotid (AD-F5-1001-503, SEQ ID NO:4),
- eine Polymerase, (Bst 2.0 Warm Start Polymerase), welche Strangverdrängungseigenschaften hat
- sowie notwendige Substrate für Polymerase (dNTP's: dATP, dCTP, dGTP, dTTP) und geeignete Puffer-Systeme (Die Reaktion wurde im Isothermal-Puffer 1x (NEB) durchgeführt).

Der Fortschritt der Reaktion wurde mittels EvaGreen detektiert bei 65°C.

Bei den eingesetzten Konzentrationsverhältnissen des ersten Primer-Oligonukleotides (5µmol/l) und des Controller-Oligonukleotides (2µmol/l) liegt der erste Primer im relativen Überschuß zum Controller-Oligonukleotid. Die Schmelztemperatur des Komplexes umfassend das erste Primer-Oligonukleotid und das Controller-Oligonukleotid betrug ca. 63°C (Tm), gemessen in der gleichen Reaktions-Lösung.

Bei der ersten Amplifikations-Reaktion wurden zwei Temperatur-Bereiche verwendet: 55°C (2 min) und 65°C (2 min). Beim Temperatur-Schritt 55°C lag das Controller-Oligonukleotid vollständig im Komplex mit dem ersten Primer-Oligonukleotid vor. Dadurch konnte das Controller-Oligonukleotid nicht an das erste Primer-Verlängerungsprodukt binden. Beim zweiten Temperatur-Schritt (65°C) konnte dieser Komplex zumindest teilweise aus dem Komplex dissoziieren, so dass in Ansatz freie, einzelsträngige Controller-Oligonukleotide verfügbar wurden. Ein solches freies Controller-Oligonukleotid kann entweder einen Komplex mit einem neuen ersten Primer-Oligonukleotid ausbilden oder sich an das erste Primer-Verlängerungsprodukt anlagern. Der Kontakt mit dem ersten Primer-Verlängerungsprodukt beginnt durch komplementäre Bindung der Sequenzsegmente des ersten Bereich des Controller-Oligonukleotides an die korrespondierende Sequenzsegmente des zweiten Primer-Bereichs, welches von der Polymerase nicht kopiert wird.

Die einzelnen Synthese-Vorgänge und Strang-Trennungs-Vorgänge umfassen im Wesentlichen folgende Prozesse:
Das erste Primer-Oligonukleotid kann dabei an das 3'-Segment der bereitgestellten Nukleinsäurekette (Start-Nukleinsäurekette 1.1) vorwiegend spezifisch binden und von der Polymerase verlängert werden (diese Reaktion verläuft vorwiegend bei 55°C). Die Synthese verläuft dabei bis zum Ende des Matrizenstranges (5'-Bereich der Start-Nukleinsäurekette). Es resultierte ein erstes Priemr-Verlängerungsprodukt. Das Controller-Oligonukleotid kann an dieses erste Primer-Verlängerungsprodukt unter Verdrängung des Matrizenstranges (hier zunächst die Start-Nukleinsäurekette) durch komplementäre Basenpaarung binden (diese Reaktion verläuft vorwiegend bei 65°C). Dabei erfolgte bei 65°C die Trennung des 3'-Segmentes des ersten Primer-Verlängerungsproduktes (welches nicht vom Controller gebunden wurde) aus dem Komplex mit dem Matrizenstrang vorwiegend Temperaturbedingt.
(die Tm dieses 3'-Segmentes lag unter 65°C). Das 3'-Segment des ersten Primer-Verlängerungsproduktes wurde dadurch einzelsträngig. Das erste Primer-Verlängerungsprodukt lag somit im Komplex mit dem Controller-Oligonukleotide vor.

Das zweite Primer-Oligonukleotid war nun in der Lage, an dieses 3'-Segment des synthetisierten ersten Primer-Verlängerungsprodukts komplementär zu binden und die Synthese des zweiten Primer-Verlängerungsproduktes durch die Polymerase zu initiieren (dieser Schritt kann dabei sowohl bei 55°C als auch bei 65°C erfolgen). Die Synthese erfolgte unter gleichzeitiger Verdrängung des Controller-Oligonukleotides aus der Bindung an das erste Primer-Verlängerungsprodukt. Die in der ersten Primer-Verlängerungsreaktion verwendete Polymerase (Bst 2.0 Warm Start) hatte eine strangverdrängende Eigenschaft. Die Synthese des zweiten Primer-Verlängerungsproduktes erfolgte bis einschließlich den ersten Bereichs des ersten Primers, welcher hierbei von der Polymerase kopiert wurde. Die Synthese des zweiten Primer-Verlängerungsproduktes wurde durch einen C3-Linker im ersten Primer-Verlängerungsproduktes terminiert, so dass der zweite Bereich des ersten Primer-Verlängerungsproduktes nicht von der Polymrease kopiert wurde. Es resultierte das zweite Primer-Verlängerungsprodukt, welches mit dem ersten Primer-Verlängerungsprodukt einen Doppelstrang bildete (Tm dieses Doppelstranges = erstes Amplifikations-Produkt betrug ca. 79°C). Beim wiederholten Erhitzen des Reaktionsgemisches auf 65°C erfolgte eine erneute Bindung des Controller-Oligonukleotides an das erste Primer-Verlängerungsprodukt, so dass nun das zweite Primer-Verlängerungsprodukt aus seiner Bindung an das erste Primer-Verlängerungsprodukt freigesetzt wurde.

Infolge der Freisetzung des zweiten Primer-Verlängerungsproduktes konnte nun dieses Produkt als Matrize dienen: das zweite Primer-Verlängerungsprodukt umfasst in seinem 3'-Segment eine Sequenz, welche komplementär zum ersten Bereich des ersten Primer ist und somit in der Lage ist, ein neues Primer-Oligonukleotid zu binden und die Synthese mittels Polymerase zu starten. Im Ergebnis erfolgten wiederholte Synthese- und Strang-Trennungsvorgänge, wobei beide synthetisierten Primer-Verlängerungsprodukte in nachfolgenden Zyklen als Matrizen auftreten konnten.

Das erste Amplifikationsprodukt konnte dabei ausgehend von der Start-Nukleinsäurekette (1.1) unter Verwendung von bereitgestellten Primern unter Mitwirkung des bereitgestellten Controller-Oligonukleotides synthetisiert werden, bis die gewünschte Menge von Kopien erreicht wurde (hier die Konzentration des ersten Amplifikations-Produktes 1.1 war gegen Ende der ersten Amplifikation ca. 0,8 - 1 µmol/l. was ca. 1000.000.000.000 Kopien in 10 µl Reaktionsansatz entspricht). Die Angabe von Kopienanzahl wurde zur Veranschaulichung ausgehend von der Konzentration abgeschätzt.

Die Reaktion wurde durch Erhitzen auf 95°C für 10 min gestoppt, wobei die erste Polymerase denaturiert wurde. Es wurde anschließend eine Schmelzkurzven-Analyse zur Bestätigung der spezifischen Amplifikation durchgeführt. Der Reaktions-Ansatz wurde eingefroren und bei Bedarf in der zweiten Amplifikation verwendet.

### Zweite Amplifikation (PCR)

Nach Abschluss ersten Amplifikation wurde ein Anteil des ersten Reaktionsansatzes in die zweite Amplifikation gegeben. Dabei diente das während der ersten Amplifikation synthetisierte erste Amplifikations-Fragment (1.1) als zweite Start-Nukleinsäurekette (2.1) für die zweite Amplifikation.

Es wurden mehrere Verdünnungs-Stufen des ersten abgeschlossenen Amplifiktions-Ansatzes in die zweite Amplifikations-Reaktion gegeben, welche in einer Verdünnngs-Reihe resultierten.

Es ist anzumerken, dass keine Aufreinigung von gebildeten ersten Amplifikations-Fragmenten (1.1) erfolgte, sondern lediglich eine Verdünnung, so dass alle nicht verbrauchten Komponenten des ersten Amplifikations-Systems - wenn auch in verdünnter Form - auch im zweiten Amplifikations-Schritt vorlagen.

Die verwendeten PCR-Reaktionsbedingungen (es wurden drei Temperaturbereiche verwendet von 55°C (1 min) und 72°C (3 min) und 95°C (20 sec)) ermöglichten die Durchführung einer Standard-PCR (zweite Amplifikation).

Das zweite Amplifikations-System umfasste jeweils folgende Komponenten:
- Ein drittes Primer-Oligonukleotid,
- ein viertes Primer-Oligonukleotid,
- eine Polymerase, (Taq Polymerase), welche eine thermostabile Polymerase ist,
- sowie notwendige Substrate für die Polymerase (dNTP's: dATP, dCTP, dGTP, dTTP) und geeignete Puffer-Systeme.

Dabei wurden für eine PCR typische Konzentrationen von einzelnen Komponenten verwendet: PCR-Primer in Endkonzentrationen von je 0,5 µmol/l, und die Taq-Polymerase in Konzentation (1.100 Verdünnung, entsprach ca. 1 Unit / Reaktion) und dNTPs (A, G,C,T) in Konzentration von ca. 200µmol/l. Die Reaktion wurde im Isothermal-Puffer 1x (NEB) durchgeführt.

Die zweite Amplifikation erfolgte unter Verwendung dieses ersten Amplifikations-Fragmentes als Start-Nukleinsäurekette (2.1) und unter Verwendung eines zweiten Amplifikations-Systems. Der Fortschritt der Synthese wurde mittels EvaGreen detektiert bei 72°C. Als Kontroll-Reaktion wurden NTC sowie Start-Nukleinsäurekette 1.1 verwendet, welche ebenfalls als Matrize im zweiten Amplifikations-System verwendet werden kann. Die bei der zweiten Amplifikation erforderliche Anzahl von Zyklen, bis zum Anstieg des spezifischen Signals, wurde gemessen und mit Referenz-Werten verglichen. Aus dem Vergleich der für die detektierbare Amplifikation erforderlichen Anzahl von Zyklen wurde auf die erfolgreiche Verwendung des ersten Amplifikations-Fragmentes (1.1) bei der PCR geschlossen. Das erste Amplifikationsprodukt einer Controller-Oligonukleotid-basierten ersten Amplifikationsreaktion wurde verdünnt und als Matrize (Input) unter typischen PCR-Bedingungen amplifiziert. Die Detektion im Rahmen der nachgeschalteten PCR erfolgt in diesem Beispiel mit dem interkalierenden Farbstoff EvaGreen. Durch den Vergleich mit einer Reihe von verschiedenen Kontrollansätzen zeigen wir, dass nur dann in der nachgeschalteten PCR ein Signal entsteht, wenn die Controller-Oligonukleotid-basierte Amplifikationsreaktion erfolgreich war. Aus diesem Grund kann die nachgeschaltete, klassische PCR auch als Detektions-PCR bezeichnet werden. Das Amplifikationsprodukt der Controller-Oligonukleotid-basierten Amplifikationsreaktion wurde im Wesentlichen hergestellt wie in Ausführungsbeispiel 2 beschrieben. Die Start-Nukleinsäurekette M2SF5-M001-200 (SEQ ID NO 6) (1.1) mit perfekt-match Übereinstimmung zum Controller wurde in einer Konzentration von etwa 1 pM eingesetzt. Im Ergebnis ist ein Amplifikations-Fragment zu erwarten, welches das zweite Primer-Verlängerungsprodukt mit folgender Sequenz ergibt: (SEQ ID NO 10). Die PCR wurde mit 3 unterschiedlichen Primer-Paaren ausgeführt. Die Primer-Paare erfassen unterschiedliche Sequenzbereiche des Amplifikationsproduktes.

Für die PCR wurden verwendet:
***PCR-Primer Paar 1*** bestehend aus dem dritten Primer-Oligonukleotid SeqP1F5 300-35x (SEQ ID NO 11) und dem vierten Primer-Oligonukleotid P2-4401-601 TMR (SEQ ID NO 12) Im Kontroll-Ansatz können Primer dieses Primer-Paares auch an der Start-Nukleinsäurekette 1.1 M2SF5-M001-200 (SEQ ID NO 6) binden.
***PCR-Primer Paar 2*** bestehend aus dem dritten Primer-Oligonukleotid P3F5D-600-203 (SEQ ID NO 14) und dem vierten Primer-Oligonukleotid P2-4401-601 TMR (SEQ ID NO 15). Diese beiden Primer binden auf folgende Weise am Matrizenstrang (SEQ ID NO 13). Im Kontroll-Ansatz können Primer dieses Primer-Paares auch an der Start-Nukleinsäurekette 1.1 (M2SF5-M001-200, SEQ ID NO 6) binden.
***PCR-Primer Paar 3*** bestehend aus dem dritten Primer-Oligonukleotid SeqP1F5-35-X02 (SEQ ID NO 16) und dem vierten Primer-Oligonukleotid P2-4401-601 TMR (SEQ ID NO 17). Im Kontroll-Ansatz können Primer dieses Primer-Paares auch an der Start-Nukleinsäurekette 1.1 M2SF5-M001-200 (SEQ ID NO 6) binden.

In der PCR wurde die Taq-Polymerase (NEB, Katalog# M0273S) eingesetzt. Dabei wurden pro Reaktion Polymerase-Verdünnungen von 1:100 eingesetzt. Das Amplifikationsprodukt aus einer Controller-Oligonukleotid-basierten Amplifikationsreaktion (erste Amplifikation) wurde in die PCR in Verdünnungen von 1:5.000 bis 1:500.000.000 eingesetzt. Zur Kontrolle wurden auch Reaktionsansätze untersucht, die keine Matrize zur Amplifikation enthielten (NTC = no template control = Negativkontrolle). Darüber hinaus wurde in weiteren Kontrollansätzen überprüft, ob das Controller-Oligonukleotid als Matrize für PCR-Primer dienen kann. Dazu wurde das Controller-Oligonukleotid (SEQ ID NO 4 = hier als "Controller 503" bezeichnet), das zuvor während der Controller-Oligonukleotid-basierten Amplifikation zum Einsatz kam, in Konzentrationen von 1 µmol/l bis 1 pmol/l zur Amplifikation in der PCR angeboten. In einer weiteren Kontrolle wurden definierte Mengen der Matrize M2SF5-M001-200 zur Amplifikation in der PCR angeboten (Positivkontrolle). Diese Kontrolle demonstrierte die Funktionstüchtigkeit der PCR-Primer-Paare (unter idealen Bedingungen). Zusätzlich konnte eine Probe nach einer ersten Amplifikation durch Vergleich der erhaltenen Ct-Werte mit einer Standard-Verdünnungs-Reihe der Positivkontrolle auch quantifiziert werden. Konkret wurde die Matrize M2SF5-M001-200 in Konzentrationen von 1 nmol/l bis 1 fmol/l ausgetestet.

Das dritte und das vierte Primer-Oligonukleotid wurde in Konzentationen von je 0,5 µmol/l eingesetzt. Die weiteren Reaktionsbedingungen waren 1x Isothermal Puffer (New England Biolabs, catalog # B0537S (der EvaGreen Farbstoff wurde entsprechend der Anleitung des Herstellers in Verdünnung 1:50 eingesetzt).

Die thermischen Reaktionsbedingungen wurden folgendermaßen gewählt:
- 20 Sekunden 95 °C zur Aktivierung des Systems
- 30 Zyklen mit jeweils folgendem Temperatur-Zeit-Profil
   ∘ 1 min 55 °C
   ∘ 3 min 72 °C
   ∘ 20 Sekunden 95 °C
- 10 min 72 °C zur Vereinheitlichung der Amplifikationsprodukte
- Schmelzkurvenanalyse

Die Amplifikation wurde typischerweise über 30 Zyklen, d.h. 30 x (1 min 55°C + 3 min 72°C + 20 s 95°C) = ca. 30 x 260 s = 130 min verfolgt. Die erfolgreiche Amplifikation konnte durch einen Anstieg des EvaGreen-Fluoreszenzsignals im Laufe der Zeit festgestellt werden.

### Analyse der Detektions-PCR

Zur Analyse der Detektions-PCR und zur Bewertung der entstandenen Amplifikationsprodukte wurden folgende Techniken genutzt:
- Fluoreszenzsignal eines interkalierenden Farbstoffes (EvaGreen)
- Schmelzkurvenanalyse von den entstandenen Amplifikationsprodukten

Bei der Analyse der PCR diskutieren wir ausschließlich Daten, die mit einer 1:100 Verdünnung an Taq-Polymerase erhalten wurden.

In den Kontroll-Experimenten mit Matrize (M2SF5-M001-200) konnte überprüft werden, dass alle drei gewählten PCR-Primer-Paare ein spezifisches PCR-Produkt unter verwendeten PCR-Bedinungen generieren konnten. Ebenfalls konnte überprüft werden, dass alle drei Primer-Paare nicht in der Lage waren, ausgehend vom Controller-Oligonukleotid ein Produkt zu bilden. Das war ein zu erwartendes Ergebnis, da alle verwendeten "dritte" Primer im modifizierten Anteil des Controller-Oligonukleotides binden.

Fig. 6A zeigt einen typischen zeitlichen Verlauf des EvaGreen-Fluoreszenzsignals, das bei der Amplifikation von verdünnten Controller-Oligonukleotid Amplifikationsansätzen, durch Primer-Paar 1 erhalten wurde. Auf der Y-Achse ist das Fluoreszenzsignal des EvaGreen-Farbstoffes und auf der X-Achse ist die Reaktionszeit (als Zyklusnummer) aufgetragen.

Pfeile markieren unterschiedliche Amplifikationsansätze. Zu den Pfeilen gehören folgende Ansätze:

| Position | Verdünnung des Controller-Oligonukleotid Amplifikationsansatz im Detektions-PCR-Ansatz |
|---|---|
| Pfeil 1 | ca. 1:5.000 |
| Pfeil 2 | ca. 1:50.000 |
| Pfeil 3 | ca. 1:500.000 |
| Pfeil 4 | ca. 1:5.000.000 |
| Pfeil 5 | ca. 1:50.000.000 |
| Pfeil 6 | 0 mol/l = Negativkontrolle |

Pfeil 6 markiert einen Negativkontrolle-Ansatz, der keine Matrize enthält und daher im Laufe des Experiments auch kein Amplifikationssignal generiert.

In Abhängigkeit von der gewählten Verdünnung des Controller-Oligonukleotid Amplifikationsansatzes werden zeitlich verschobene Amplifikationssignale beobachtet. Für PCR-Primer-Paar 1 können unter den gewählten Bedingungen Verdünnungen von 1:5.000 bis 1:50.000.000 gut aufgelöst werden. Eine Verdünnung von 1:50.000.000 erzeugt ein Amplifikationssignal, das sich marginal von der Negativkontrolle abhebt (vgl. Pfeil 5).

Fig. 6B zeigt die zugehörige Schmelzkurvenanalyse für die Reaktionen 1 bis 5 und macht deutlich, dass spezifische Amplifikationsprodukte entstanden sind (Tm = 82,7°C).

Fig. 6B zeigt die Schmelzkurven der Amplifikationsprodukte von Primer-Paar 1. Auf der Y-Achse ist die Ableitung des Fluoreszenzsignals gegen die Temperatur und auf der X-Achse ist die Temperatur aufgetragen. Es wurde ein einheitlicher Peak gefunden, markiert mit Pfeil 1. Der Peak bei Position 1 mit einem Schmelzpunkt in der Nähe von 82,7 °C gehört zum Amplifikationsprodukt des Primer-Paar 1. Die Schmelzkurvenanalyse weist auf eine spezifische Amplifikation hin.

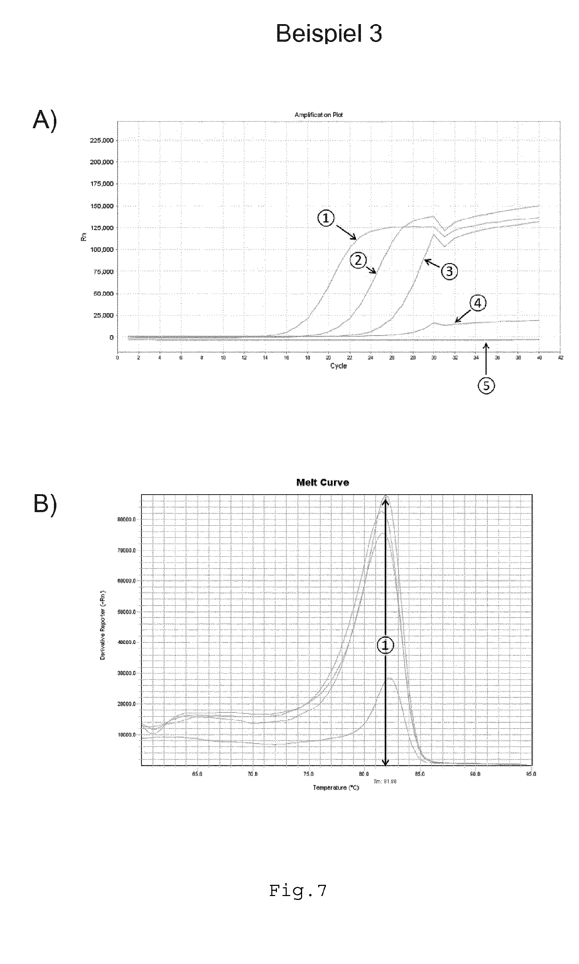

Fig. 7A zeigt einen typischen zeitlichen Verlauf des EvaGreen-Fluoreszenzsignals, das bei der Amplifikation von verdünnten Controller-Oligonukleotid Amplifikationsansätzen, durch PCR-Primer-Paar 2 erhalten wurde. Auf der Y-Achse ist das Fluoreszenzsignal des EvaGreen-Farbstoffes und auf der X-Achse ist die Reaktionszeit (als Zyklusnummer) aufgetragen. Pfeile markieren unterschiedliche Amplifikationsansätze. Zu den Pfeilen gehören folgende Ansätze:

| Position | Verdünnung des Controller-Oligonukleotid Amplifikationsansatz im Detektions-PCR-Ansatz |
|---|---|
| Pfeil 1 | ca. 1:5.000 |
| Pfeil 2 | ca. 1:50.000 |
| Pfeil 3 | ca. 1:500.000 |
| Pfeil 4 | ca. 1:5.000.000 |
| Pfeil 5 | 0 mol/l = Negativkontrolle |

Pfeil 5 markiert einen Negativkontrolle-Ansatz, der keine Matrize enthält und daher im Laufe des Experiments auch kein Amplifikationssignal generiert.

In Abhängigkeit von der gewählten Verdünnung des Controller-Oligonukleotid Amplifikationsansatzes werden zeitlich verschobene Amplifikationssignale beobachtet. Für PCR-Primer-Paar 2 können unter den gewählten Bedingungen Verdünnungen von 1:5.000 bis 1:5.000.000 gut aufgelöst werden. Eine Verdünnung von 1:5.000.000 erzeugt ein Amplifikationssignal, das sich gerade von der Negativkontrolle abhebt (Pfeil 4).

Fig. 7B zeigt die zugehörige Schmelzkurvenanalyse für die Reaktionen 1 bis 4 und macht deutlich, dass spezifische Amplifikationsprodukte entstanden sind.

Fig. 7B zeigt die Schmelzkurven der Amplifikationsprodukte von PCR-Primer-Paar 2 aus Fig. 7A. Auf der Y-Achse ist die Ableitung des Fluoreszenzsignals gegen die Temperatur und auf der X-Achse ist die Temperatur aufgetragen. Es wurde ein einheitlicher Peak gefunden, markiert mit Pfeil 1. Der Peak bei Position 1 mit einem Schmelzpunkt in der Nähe von 81,9 °C gehört zum Amplifikationsprodukt des PCR-Primer-Paar 2. Die Schmelzkurvenanalyse weist auf eine spezifische Amplifikation hin.

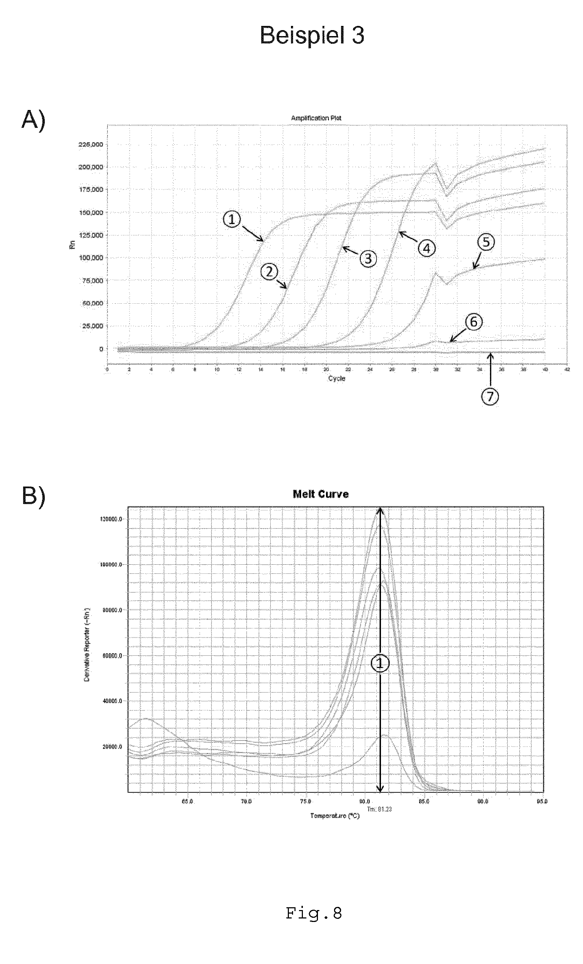

Fig. 8A zeigt einen typischen zeitlichen Verlauf des EvaGreen-Fluoreszenzsignals, das bei der Amplifikation von verdünnten Controller-Oligonukleotid Amplifikationsansätzen, durch PCR-Primer-Paar 3 erhalten wurde. Auf der Y-Achse ist das Fluoreszenzsignal des EvaGreen-Farbstoffes und auf der X-Achse ist die Reaktionszeit (als Zyklusnummer) aufgetragen. Pfeile markieren unterschiedliche Amplifikationsansätze. Zu den Pfeilen gehören folgende Ansätze:

| Position | Verdünnung des Controller-Oligonukleotid Amplifikationsansatz im Detektions-PCR-Ansatz |
|---|---|
| Pfeil 1 | ca. 1:5.000 |
| Pfeil 2 | ca. 1:50.000 |
| Pfeil 3 | ca. 1:500.000 |
| Pfeil 4 | ca. 1:5.000.000 |
| Pfeil 5 | ca. 1:50.000.000 |
| Pfeil 6 | ca. 1:500.000.000 |
| Pfeil 7 | 0 mol/l = Negativkontrolle |

Pfeil 7 markiert einen Negativkontrolle-Ansatz, der keine Matrize enthält und daher im Laufe des Experiments auch kein Amplifikationssignal generiert. In Abhängigkeit von der gewählten Verdünnung des Controller-Oligonukleotid Amplifikationsansatzes werden zeitlich verschobene Amplifikationssignale beobachtet. Für Primer-Paar 3 können unter den gewählten Bedingungen Verdünnungen von 1:5.000 bis 1:500.000.000 gut aufgelöst werden. Eine Verdünnung von 1:500.000.000 erzeugt ein Amplifikationssignal, das sich gerade von der Negativkontrolle abhebt (vgl. Pfeil 6 und 7).

Fig. 8B zeigt die zugehörige Schmelzkurvenanalyse für die Reaktionen 1 bis 6 und macht deutlich, dass spezifische Amplifikationsprodukte entstanden sind.

Fig. 8B zeigt die Schmelzkurven der Amplifikationsprodukte von PCR-Primer-Paar 3 aus Fig. 8A. Auf der Y-Achse ist die Ableitung des Fluoreszenzsignals gegen die Temperatur und auf der X-Achse ist die Temperatur aufgetragen. Es wurde ein einheitlicher Peak gefunden, markiert mit Pfeil 1. Der Peak bei Position 1 mit einem Schmelzpunkt in der Nähe von 81,2 °C gehört zum Amplifikationsprodukt des PCR-Primer-Paar 3. Die Schmelzkurvenanalyse weist auf eine spezifische Amplifikation hin.

Durch Analyse der Fluoreszenzsignale im zeitlichen Verlauf der Amplifikation (Amplification Plots) und durch die Schmelzkurvenanalyse am Ende der Amplifikation wurde nachgewiesen, dass die PCR-Primer-Paare 1 bis 3 jeweils in der Lage waren, verdünnte Amplifikationsprodukte, die aus der ersten Amplifikationsreaktion hervorgegangen waren, als Matrize zu nutzen. In Abhängigkeit von der gewählten Verdünnung entstehen zeitlich verzögerte Amplifikationssignale, welche für eine Verdünnngsreihe typisch sind. Durch die hier gewählten PCR Bedingungen können 5.000.000-fach verdünnte Amplifikationsprodukte nachgewiesen werden. Das Primer Paar 3 weist die höchste Empfindlichkeit auf und kann sogar noch Amplifikationsprodukte bei 500.000.000-facher Verdünnung detektieren.

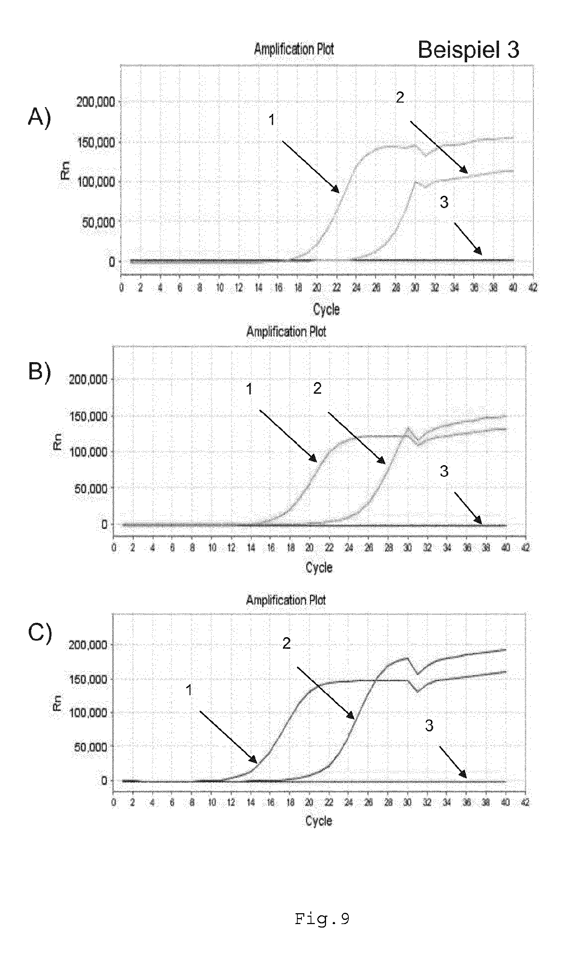

Fig. 9 zeigt Kontroll-Experimente mit Matrize (M2SF5-M001-200), welche in 1 nmol/l (Pfeil 1) und 10 pmol/l (Pfeil 2) eingesetzt wurde, sowie NTC-Kontrollen (Pfeil 3) (Fig. 9A stellt Ergebnisse für PCR-Primer Paar 1 dar; Fig. 9B stellt Ergebnisse für PCR-Primer Paar 2 dar; Fig. 9C stellt Ergebnisse für PCR-Primer Paar 3 dar).

Aus dem Vergleich der Zeit des Anstieges des Fluoreszenzsignals zwischen Kontroll-Experimenten (Matrize) und der durchgeführten zweiten Amplifikation ausgehend von ersten Amplifikations-Fragmenten ist ersichtlich, dass die erste Amplifiaktion eine Vermehrung von Amplifikations-Fragmenten bewirkte, welche in der zweiten Amplifikation als Matrize verwendet werden konnten.

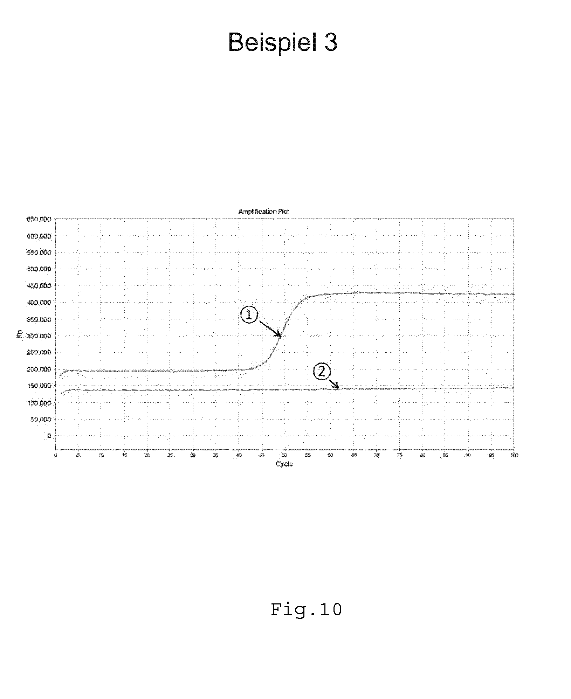

Fig. 10 zeigt den Verlauf der ersten Amplifikation (vor PCR).

Man erkennt einen Signal-Anstiegt, welcher als Anreicherung der Kopie-Anzahl in der ersten Amplifikation gedeutet wurde. Pfeil 1 entsprach einem Ansatz mit einer Start-Nukleinsäurekette 1.1. Pfeil 2 = NTC Kontrolle.

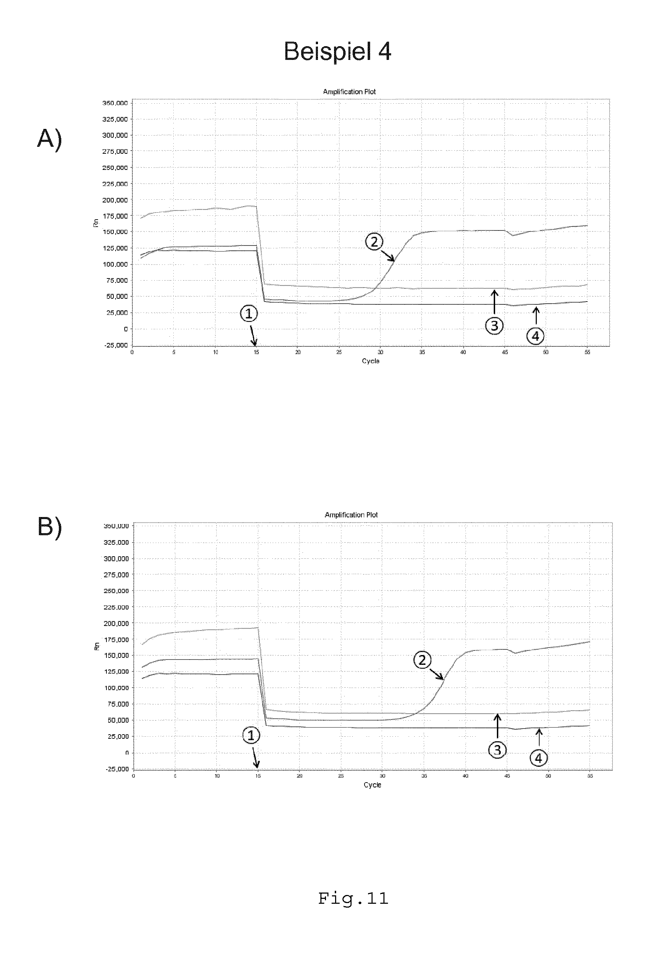

### Beispiel 4 (Fig. 11 - 14):

### Kombination von der ersten Amplifikation und der zweiten Amplifikation im gleichen Ansatz (homogenen Assay)

In diesem Ausführungsbeispiel demonstrieren wir wie die Controller-Oligonukleotid-basierte Amplifikationsreaktion mit einer nachgeschalteten, klassischen PCR in einem homogenen Assay-Format kombiniert werden kann. Hierbei nutzen wir die hochselektive Controller-Oligonukleotid-basierte Amplifikationsreaktion in der frühen Amplifikationsphase und schalten danach zur Amplifikation und Detektion der Amplifikationsprodukte auf eine klassische PCR um. Im Gegensatz zu Ausführungsbeispiel X (= heterogenes Assay-Format) erfolgt die Kombination in diesem Ausführungsbeispiel in einem homogenen Assay-Format. Alle Assay-Komponenten liegen bereits beim Reaktionsstart vor, das Umschalten zwischen Controller-Oligonukleotid-basierter Amplifikation und klassischer PCR erfolgt durch eine Änderung der Temperaturführung im Laufe der Reaktion.

Zuerst wurde das erste Amplifikations-Produkt 1.1 ausgehend von einer einzelsträngigen Start-Nukleinsäurkette (1.1) mittels Controller-Oligonukleotid-basierter Amplifikationsreaktion für 15 bis 30 Zyklen amplifiziert, danach erfolgten 30 klassische PCR-Zyklen, um das voramplifizierte Produkt weiter zu amplifizieren und zu detektieren. Zur Detektion verwenden wir in diesem Beispiel den interkalierenden Farbstoff EvaGreen.

Das erste Amplifikations-System umfasste folgende Komponenten:
- Ein erstes Primer-Oligonukleotid (P1F5-200-AE205, SEQ ID NO 19),
- ein zweites Primer-Oligonukleotid (P2G3-5270-7063, SEQ ID NO 8),
- ein Controller-Oligonukleotid (AD-F5-1001-503, SEQ ID NO 4);
- eine Polymerase, (Bst 2.0 Warm Start Polymerase), welche Strangverdrängungseigenschaften hat;
- sowie notwendige Substrate für die Polymerase (dNTP's: dATP, dCTP, dGTP, dTTP) und geeignete Puffer-Systeme (Die Reaktion wurde im Isothermal-Puffer 1x (NEB) durchgeführt).

Das zweite Amplifikations-System umfasste jeweils folgende Komponenten:
- Ein drittes Primer-Oligonukleotid (P3F5D-600-201 [SEQ ID NO 20]; P3F5D-600-202 [SEQ ID NO 21]; P3F5D-600-203 [SEQ ID NO 22]; oder P3F5D-600-204 [SEQ ID NO 23]),
- ein viertes Primer-Oligonukleotid
- eine Polymerase, (Taq Polymerase), welche eine thermostabile Polymerase ist
- sowie notwendige Substrate für die Polymerase (dNTP's: dATP, dCTP, dGTP, dTTP) und geeignete Puffer-Systeme.

Beide Amplifikations-Systeme lagen vor Beginn der ersten Amplifikation zusammen vor.

In diesem Beispiel wurde das zweite Primer-Oligonukleotid für beide Amplifikationen verwendet. Das vierte Primer-Oligonukleotid ist somit identisch mit dem zweiten Primer-Oligonukleotid. Das dritte Primer-Oligonukleotid wurde variiert. In diesem Beispiel präsentieren wir 4 unterschiedliche Primer, welche jeweils einzeln pro Ansatz als "das dritte Primer-Oligonukleotid" verwendet wurden.

Das jeweilige dritte Primer-Oligonukleotid unterscheidet sich vom ersten Primer-Oligonukleotid. Somit umfasst jeder Reaktions-Ansatz drei Primer: ein erstes Primer-Oligonukleotid und ein zweites Primer-Oligonukleotid (als Komponenten des ersten Amplifikations-Systems) und das dritte-Primer-Oligonukleotid, welches als spezifischer Primer des zweiten Amplifikations-Systems verwendet wurde. Die Rolle des vierten Primers übernahm der zweite Primer des ersten Amplifikations-Systems, welches für die nachgeschaltete PCR-Reaktion ebenfalls geeignet war.

Folgende Start-Nukleinsäurekette Matrize (1.1) M2SF5-M001-200 (SEQ ID NO 18) wurde als einzelsträngige Matrize verwendet mit einer Sequenzzusammensetzung, welche zu einem ersten Primer-Verlängerungsprodukt mit einer Perfekt-Match-Übereinstimmung mit dem Controller-Oligonukleotid führt. Die Start-Nukleinsäurekette 1.1 (Matrize) wurde in Konzentrationen von 1 pmol/l eingesetzt. Primer 1 wurde mit 5 µmol/l, das Controller-Oligonukleotid mit 2 µmol/l, Primer 2 mit 1 µmol/l und Primer 3 mit 0,5 µmol/l eingesetzt. Zur Amplifikation befanden sich 2 Polymerasen im Ansatz Taq-Polymerase (NEB, Katalog# M0273S?) in einer Verdünnung 1-zu-100 und Bst 2.0 Warm Start Polymerase (NEB, Katalog# M0538S?) in einer Verdünnung 1-zu-200. In einer Kontroll-Reaktion wurde kein P3-Primer eingesetzt. In weiteren Kontroll-Reaktionen wurde nur BST-Polymerase, aber keine Taq-Polymerase eingesetzt.

Die weiteren Reaktionsbedingungen waren 1x Isothermal Puffer (New England Biolabs, catalog # B0537S), wobei der EvaGreen Farbstoff entsprechend der Anleitung des Herstellers in Verdünnung 1:50 eingesetzt wurde.

Die thermischen Reaktionsbedingungen wurden nach folgendem Profil gewählt:
- 2 min 65 °C zur Aktivierung des Systems
- 15 bzw. 30 Zyklen mit folgendem Temperatur-Zeit-Profil (Controller-Oligonukleotid-basierte Amplifikationsreaktion)
   ∘ 2 min 55 °C
   ∘ 2 min 65 °C
- 20 Sekunden 95 °C zur Aktivierung des Systems
- 30 Zyklen mit folgendem Temperatur-Zeit-Profil (Detektions-PCR)
   ∘ 1 min 55 °C
   ∘ 3 min 72 °C
   ∘ 20 Sekunden 95 °C
- 10 min 72 °C zur Vereinheitlichung der Amplifikationsprodukte
- Schmelzkurvenanalyse

Die Gesamt-Amplifikation wurde typischerweise über 45-60 Zyklen, d.h.:
Erste Amplifikation: (15 bzw. 30) x (2 min 55°C + 2 min 65°C) = (15 bis 30) x 4 min = 60 bis 120 min, zuzüglich zweite Amplifikation (PCR): 30 x (1 min 55°C + 3 min 72°C + 20 s 95°C) = 30 x 260 s = 130 min verfolgt. Dies entspricht einer Gesamtzeit von 190 bis 250 min.

Die erfolgreiche Amplifikation konnte durch einen Anstieg des EvaGreen-Fluoreszenzsignals im Laufe der Zeit festgestellt werden.

### Analyse der Kombination aus Controller-Oligonukleotid-basierter Amplifikationsreaktion und Detektions-PCR.

Zur Analyse und zur Bewertung der entstandenen Amplifikationsprodukte wurden folgende Techniken genutzt:
- Fluoreszenzsignal eines interkalierenden Farbstoffes (EvaGreen)
- Schmelzkurvenanalyse von den entstandenen Amplifikationsprodukten

Die erste Teil-Amplifikation (mit 15 Zyklen oder mit 30 Zyklen) führt zu einer Anreicherung der Nukleinsäureketten (das erste Amplifikations-Fragment 1.1), welche als Matrizen in der nachgeschalteten PCR verwendet werden können. Dadurch braucht die PCR weniger Zyklen, um detektierbare Mengen an PCR-Produkt zu generieren. Durch die Wahl der Anzahl an Zyklen in den beiden Amplifikationsphasen kann der Verstärkungsanteil an der Gesamtverstärkung für die beiden Amplifikationsreaktionen eingestellt werden. Durch den Vergleich mit einer Reihe von verschiedenen Kontrollansätzen zeigen wir, dass durch die Verwendung einer ersten Amplifikation, die Zyklus-Zahl der zweiten Amplifikation (PCR) reduziert werden konnte, bis ein detektierbares Signal entstand (Ct). Diese Minderung der Zyklus-Anzahl der PCR erfolgte nur dann, wenn die erste Amplifikation (Controller-Oligonukleotid-basierte Amplifikationsreaktion) erfolgreich war. Die zweite Amplifikation erfolgte dann ausgehend von ersten Amplifikationsprodukten bis zu Detektion. Aus diesem Grund kann die nachgeschaltete, klassische PCR auch als Detektions-PCR bezeichnet werden. Weiterhin zeigen wir, dass eine Kombination des ersten und des zweiten Amplifikations-Systems in einem Ansatz möglich ist, so dass ein homogenes Assay Format durchgeführt werden konnte.

Fig. 11A zeigt einen typischen Verlauf des EvaGreen-Fluoreszenzsignals im Laufe der kombinierten Amplifikation von Matrize M2SF5-M001-200 mit Primer P3F5D-600-201. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 15 kommt die Controller-Oligonukleotid-basierte Amplifikationsreaktion zum Einsatz. Ab Zyklus 15 bis Zyklus 45 kommt die klassische PCR (Detektions-PCR) zum Einsatz. Der Umschaltzeitpunkt (Ende Zyklus 15) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-201 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

Fig. 11B zeigt einen typischen Verlauf des EvaGreen-Fluoreszenzsignals im Laufe der kombinierten Amplifikation von Matrize M2SF5-M001-200 mit Primer P3F5D-600-202. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 15 kommt die Controller-Oligonukleotid-basierte Amplifikationsreaktion zum Einsatz. Ab Zyklus 15 bis Zyklus 45 kommt die klassische PCR (Detektions-PCR) zum Einsatz. Der Umschaltzeitpunkt (Ende Zyklus 15) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-202 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

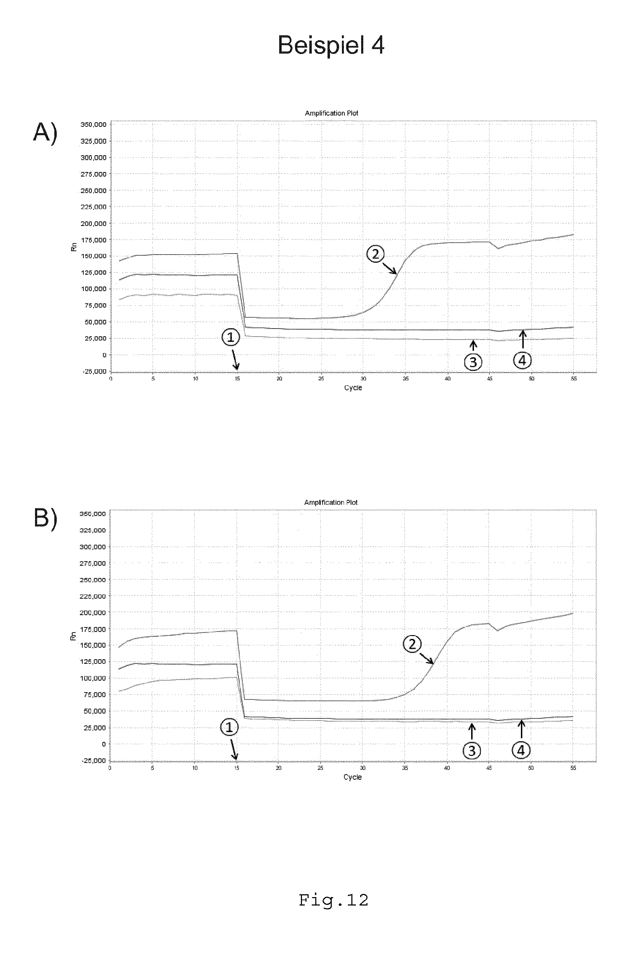

Fig. 12A zeigt einen typischen Verlauf des EvaGreen-Fluoreszenzsignals im Laufe der kombinierten Amplifikation von Matrize M2SF5-M001-200 mit Primer P3F5D-600-203. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 15 kommt die Controller-Oligonukleotid-basierte Amplifikationsreaktion zum Einsatz. Ab Zyklus 15 bis Zyklus 45 kommt die klassische PCR (Detektions-PCR) zum Einsatz. Der Umschaltzeitpunkt (Ende Zyklus 15) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-203 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

Fig. 12B zeigt einen typischen Verlauf des EvaGreen-Fluoreszenzsignals im Laufe der kombinierten Amplifikation von Matrize M2SF5-M001-200 mit Primer P3F5D-600-204. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 15 kommt die Controller-Oligonukleotid-basierte Amplifikationsreaktion zum Einsatz. Ab Zyklus 15 bis Zyklus 45 kommt die klassische PCR (Detektions-PCR) zum Einsatz. Der Umschaltzeitpunkt (Ende Zyklus 15) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-204 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

Es zeigt sich, dass eine Kombination von Controller-Oligonukleotid-basierter Amplifikationsreaktion und (klassischer) Detektions-PCR mit jeder der 4 Primer-3-Varianten (P3F5D-600-201 bis P3F5D-600-204) im homogenen Assay-Format gelingt. Dabei ist der kombinierte Einsatz von BST- und Taq-Polymerase wichtig. Ebenso wichtig ist der dritte Primer, ohne den keine Amplifikationssignale zu detektieren waren. Die Primer-3-Varianten verhalten sich durchaus individuell, was an den unterschiedlichen Zeitpunkten zu erkennen ist, zu denen sich das Amplifikationssignal von der Basislinie abzuheben beginnt. Je früher ein Amplifikationssignal in der Detektions-PCR zu erkennen ist, um so effizienter ist die jeweilige Primer-3-Variante in der Detektion von zuvor entstandenen Controller-Oligonukleotid-basierten Amplifikationsprodukten.

Nachfolgend wurde gezeigt, dass eine Verdopplung der Zyklusanzahl der Controller-Oligonukleotid-basierten Amplifikationsreaktion in der frühen Amplifikationsphase zu qualitativ sehr ähnlichen Ergebnissen führt.

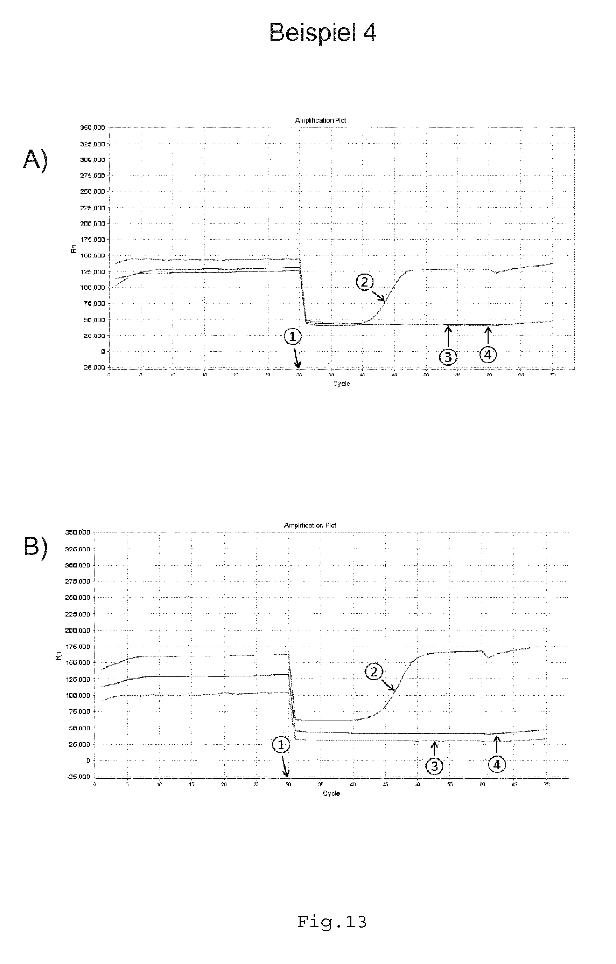

Fig. 13A zeigt einen typischen Verlauf des EvaGreen-Fluoreszenzsignals im Laufe der kombinierten Amplifikation von Matrize M2SF5-M001-200 mit Primer P3F5D-600-201. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 30 kommt die Controller-Oligonukleotid-basierte Amplifikationsreaktion zum Einsatz. Ab Zyklus 31 bis Zyklus 60 kommt die klassische PCR (Detektions-PCR) zum Einsatz. Der Umschaltzeitpunkt (Ende Zyklus 30) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-201 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

Fig. 13B zeigt einen typischen Verlauf des EvaGreen-Fluoreszenzsignals im Laufe der kombinierten Amplifikation von Matrize M2SF5-M001-200 mit Primer P3F5D-600-203. Auf der Y-Achse ist das Fluoreszenzsignal des EvaGreen-Farbstoffes und auf der X-Achse ist die Reaktionszeit (als Zyklusnummer) aufgetragen. In den Zyklen 1 bis 30 kommt die Controller-Oligonukleotid-basierte Amplifikationsreaktion zum Einsatz. Ab Zyklus 31 bis Zyklus 60 kommt die klassische PCR (Detektions-PCR) zum Einsatz. Der Umschaltzeitpunkt (Ende Zyklus 30) ist mit Pfeil 1 markiert. Pfeil 2 markiert einen Amplifikationsansatz, in dem die Matrize M2SF5-M001-200 ausgehend von 1 pmol/l Startkonzentration durch BST- und Taq-Polymerase amplifiziert wird. Pfeil 3 markiert einen Kontrollansatz, der nur BST-Polymerase enthält. Eine Amplifikation unterbleibt, da die hitzeempfindliche BST-Polymerase ab Zyklus 16 durch das Temperatur-Profil in der Detektions-PCR denaturiert wird. Bereits entstandene Amplifikationsprodukte aus der Controller-Oligonukleotid-basierten Amplifikationsreaktion bleiben unter der Detektionsschwelle von EG verborgen. Pfeil 4 markiert einen weiteren Kontrollansatz, bei dem auf die Zugabe des Primers P3F5D-600-203 verzichtet wurde. Ein Amplifikationssignal wird nun nicht mehr beobachtet, da ohne den dritten Primer keine exponentielle Amplifikation in der Detektions-PCR möglich ist. Bereits entstandene Amplifikationsprodukte aus der Controller-Oligonukleotid-basierten Amplifikationsreaktion bleiben unter der Detektionsschwelle von EG verborgen.

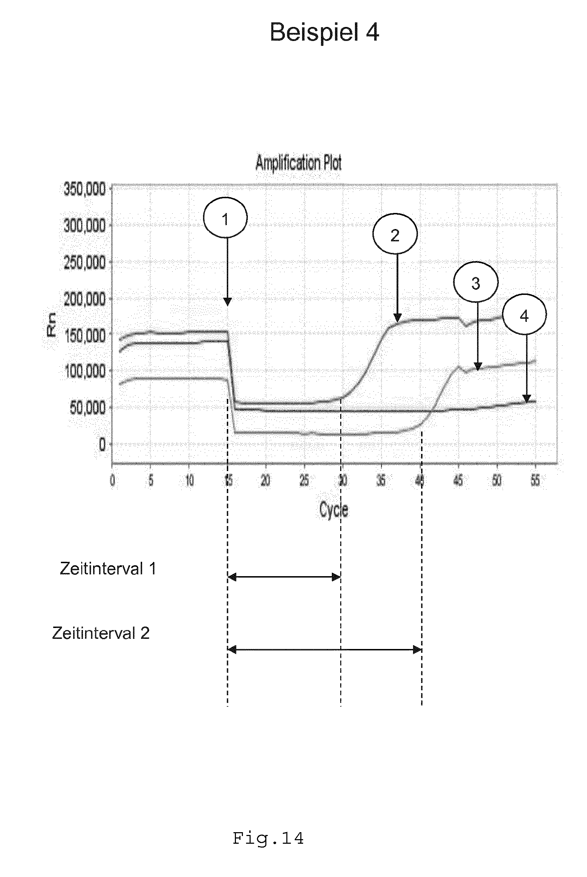

Fig. 14 zeigt einen Vergleich von Zeitintervalen zwischen einer Kombinierten Amplifikation (umfassend die erste und die zweite Amplifikation) vs. PCR alleine (nur die zweite Amplifikation). Die erste Amplifikation wurde wie oben dargestellt ausgeführt. In der zweiten Reaktion war als drittes Primer-Oligonukleotid (P3F5D-600-203) verwendet. Die Ansätze wurden wie oben beschrieben pipettiert und die Reaktionen durchgeführt. Wie oben dargestellt wurde bei einem Kombinierten Ansatz zunächst eine Controller-Abhängige Amplifikation durchgeführt (hier 15 Zyklen) anschließend wurde auf PCR umgeschaltet (Pfeil 1). Verglichen wurden die Zeitintervale 1 und 2. Pfeil 2 zeigt den Verlauf der Kombinierten Amplifikation (Bst und Taq Polymerase waren beide im Ansatz). Im Ansatz befand sich 1 pmol/l Start-Nukleinsäurekette (Matrize) vor dem Beginn der ersten Amplifikation. Der Anstieg des Fluoreszenzsignals erfolgt nach einem Zeitinterval 1. Pfeil 3 zeigt den Verlauf eines Kontroll-Ansatzes, bei welchem die Bst weggelassen wurde, so dass die erste Amplifikations-Reaktion "still-gelegt wurde" (zwar befanden sich alle Komponenten des ersten Amplifikations-System im Ansatz, dennoch fehlte es an der richtigen Polymerase, in diesem Fall Bst 2.0 Polymerase). Im Ansatz befand sich 1 pmol/l Start-Nukleinsäurekette (Matrize) vor dem Beginn der ersten Amplifikation. Der Anstieg des Fluoreszenzsignals erfolgt nach einem Zeitinterval 2. Die Taq Polymerase kann zwar ausgehend von der gleichen Template auch ein Produkt sythetisieren, die PCR alleine braucht aber mehr Zyklen dafür. Pfeil 4 zeigt den Verlauf von Kombiniertem Ansatz ohne Start-Nukleinsäurekette 1.1 (ohne Matrize =NTC-Kontrolle).

Insgesamt sieht man, dass durch die Vermehrung von Amplifikations-Fragmenten 1.1 während der ersten Amplifikation, welche anschließend in der PCR als Start-Nukleinsäurekette 2.1 (Matrize) dienen können, erfolgt die Erzeugung von PCR-Fragmenten die Reaktion im Ansatz 2 insgesamt schneller: die PCR braucht weniger Zyklen, bis eine detektierbare Menge des zweiten Amplifikationsproduktes (2.1) hergestellt wurde. ZeitInterval 1 ist kürzer als Zeitinterval 2.

Fig. 14 zeigt einen Vergleich von Zeitintervalen zwischen einer Kombinierten Amplifikation (umfassend die erste und die zweite Amplifikation) vs. PCR alleine (nur die zweite Amplifikation). Die erste Amplifikation wurde wie oben dargestellt ausgeführt. In der zweiten Reaktion war als drittes Primer-Oligonukleotid (P3F5D-600-203) verwendet. Die Ansätze wurden wie oben beschrieben pipettier und Reaktionen durchgeführt. Wie oben dargestellt wurde bei einem Kombinierten Ansatz zunächst eine Controller-Abhängige Amplifikation durchgeführt (hier 30 Zyklen) anschließend wurde auf PCR umgeschaltet (Pfeil 1). Verglichen wurden die Zeitintervale 3 und 4. Pfeil 2 zeigt den Verlauf der Kombinierten Amplifikation (Bst und Taq Polymerase waren beide im Ansatz). Im Ansatz befand sich 1 pmol/l Start-Nukleinsäurekette (Matrize) vor dem Beginn der ersten Amplifikation. Der Anstieg des Fluoreszenzsignals erfolgt nach einem Zeitinterval 3. Pfeil 3 zeigt den Verlauf eines Kontroll-Ansatzes, bei welchem die Bst weggelassen wurde, so dass die erste Amplifikations-Reaktion "still-gelegt wurde" (zwar befanden sich alle Komponenten des ersten Amplifikations-System im Ansatz, dennoch fehlte es an der richtigen Polymerase, in diesem Fall Bst 2.0 Polymerase). Im Ansatz befand sich 1 pmol/l Start-Nukleinsäurekette (Matrize) vor dem Beginn der ersten Amplifikation. Der Anstieg des Fluoreszenzsignals erfolgt nach einem Zeitinterval 4. Die Taq Polymerase kann zwar ausgehend von der gleichen Template auch ein Produkt sythetisieren, die PCR alleine braucht aber mehr Zyklen dafür. Pfeil 4 zeigt den Verlauf von Kombiniertem Ansatz ohne Start-Nukleinsäurekette 1.1 (ohne Matrize =NTC-Kontrolle).

Insgesamt sieht man, dass durch die Vermehrung von Amplifikations-Fragmenten 1.1 während der ersten Amplifikation, welche anschließend in der PCR als Start-Nukleinsäurekette 2.1 (Matrize) dienen können, erfolgt die Erzeugung von PCR-Fragmenten die Reaktion im Ansatz 2 insgesamt schneller: die PCR braucht weniger Zyklen, bis eine detektierbare Menge des zweiten Amplifikationsproduktes (2.1) hergestellt wurde. ZeitInterval 3 ist kürzer als Zeitinterval 4.

## Patentansprüche

1. Ein Verfahren zur Amplifikation einer Nukleinsäure, wobei
a) eine Probe, die ein erstes Nukleinsäurepolymer umfassend mindestens eine erste Zielsequenz M [und die zu M (revers) komplementäre Sequenz M'], wobei M in 5'-3'-Orientierung in unmittelbarer Folge die Sequenzabschnitte MPL, MS und MPR umfasst, in einem ersten Amplifikationsschritt mit folgenden Komponenten in Kontakt gebracht wird:
b) eine erste matrizenabhängige Nukleinsäurepolymerase, insbesondere eine DNA-Polymerase, sowie Substrate der matrizenabhängigen Nukleinsäurepolymerase (insbesondere Ribonukleosid-triphosphate oder Desoxyribonukleosid-triphosphate) und geeignete Kofaktoren.
c) ein erster linker Oligonukleotidprimer PL1, welcher (im Wesentlichen) identisch mit MPL ist;
d) ein erster rechter Oligonukleotidprimer PR1, der in 5'-3'-Orientierung in unmittelbarer Folge die Sequenzabschnitte PCR und PMR umfasst, wobei PMR die zu MPR komplementäre [hybridisierende] Sequenz aufweist [im Wesentlichen sequenzspezifisch binden kann] und der Sequenzabschnitt PCR nicht an M [oder eine in Bezug auf die Sequenz M in 3' unmittelbar an MPR anschließende Sequenz] binden kann, und
wobei PR1 (insbesondere im Abschnitt PCR) modifizierte Nukleotidbausteine umfasst, so dass PCR nicht als Matrize für die Aktivität der ersten matrizenabhängigen Nukleinsäurepolymerase dienen kann; und
e) ein Controller-Oligonukleotid CR, das in 5'-3'-Orientierung die Sequenzabschnitte CSR, CPR und CCR in unmittelbarer Folge umfasst, wobei eine Nukleinsäuresequenz am 3'Ende von CSR mit einer Länge von mindestens 10 Nukleotiden, insbesondere die gesamte Nukleoinsäuresequenz von CSR identisch zu einem Abschnitt von MS, insbesondere einem Abschnitt am 3'Ende von MS, ist, welcher in 5' von MPR liegt [und bei Initiation der Polymerase von PR zuerst abgelesen wird, CSR also mindestens an einen Teil des Verlängerungsprodukts des Primers PR1 binden kann], CPR komplementär zu PMR ist und CCR eine zu PCT komplementäre Sequenz zu PCR umfasst und an PCR binden kann,
und wobei CR in CSR und / oder CPR modifizierte Nukleotidbausteine umfasst, so dass CSR nicht als Matrize für die Aktivität der matrizenabhängigen Nukleinsäurepolymerase dienen kann; und
wobei die Probe in einem zweiten Amplifikationsschritt mit folgenden Komponenten in Kontakt gebracht wird:
a) ein zweiter linker Oligonukleotidprimer PL2, welcher zu einem ersten Sekundärprimerbindungsbereich MPL2 identisch ist, und
b) ein zweiter rechter Oligonukleotidprimer PR2, welcher zu einem Bereich MPR2 komplementär ist,
wobei MPL2 und MPR2 von M umfasst werden und das 3'-Ende von MPL2 [mindestens 5, 10 oder 20 Positionen] in 5'-Richtung des 5'-Endes von MPR2 angeordnet ist, (insbesondere MPL2 mit MPL und /oder MPR2 mit MPR identisch ist).

2. Das Verfahren zur Amplifikation einer Nukleinsäure gemäß Anspruch 1, wobei ein erstes Primer-Verlängerungsprodukt PR1' erhalten wird, welches in 5'-3'-Orientierung neben den Sequenzbereichen PCR und PMR einen synthetisierten Bereich PSR umfasst, der im Wesentlichen komplementär zur Zielsequenz M in dem in 5' an MPR angrenzenden Bereich ist, und insbesondere eine zu MPL Im Wesentlichen komplementäre Sequenz umfasst, und ein zweites Primer-Verängerungsprodukt PL1'erhalten wird, welches neben dem Sequenzbereich MPL einen synthetisierten Bereich PSL umfasst, der im Wesentlichen identisch zur Zielsequenz M im an MPL in 3' angrenzenden Bereich ist, und insbesondere eine zu MPR im Wesentlichen identische Sequenz umfasst,
und wobei
- entweder die Reaktionsbedingungen des ersten Amplifikationsschrittes so gewählt sind, und/oder
- die Längen und Schmelztemperaturen von PCR und ggf. MS so gewählt sind,
dass PR1' mit M oder mit PL1'einen Doppelstrang bilden kann, [PL1' mit M' oder PR1' einen Doppelstrang bilden kann,] [PR1' mit PL1'einen Doppelstrang bilden kann], und PR1' mit C einen Doppelstrang bilden kann und die Bildung des Doppelstranges aus PR1' und C gegenüber der Bildung des Doppelstrangs aus PR1' mit M [mindestens im Bereich von MPR] bevorzugt ist (bzw. zu Trennung von PR1' von M oder zu Trennung von PR1' von PL1' führen kann), und insbesondere ein drittes Primer-Verlängerungsprodukt PL2' erhalten wird, welches einen synthetisierten Bereich aufweist, der zum Bereich MPR2 identisch ist, und ein viertes Primer-Verlängerungsprodukt PR2' erhalten wird, der einen synthetisierten Bereich aufweist, der zum Bereich MPL2 komplementär ist.

3. Das Verfahren zur Amplifikation einer Nukleinsäure gemäß Anspruch 2, wobei
- entweder die Reaktionsbedingungen des ersten Amplifikationsschrittes so gewählt sind, und/oder
- die Längen und Schmelztemperaturen von PCR und ggf. MS und/oder die Positionen von MPL und MPR so gewählt sind,
dass sich in Abwesenheit des Controller-Oligonukleotides CR das erste Primer-Verlängerungsprodukt PR1' nicht vom zweiten Primer-Verlängerungsprodukt PL1' trennt.

4. Das Verfahren gemäß einem der Ansprüche 1 bis 3, wobei PL2 und/oder PR2 [unmittelbar] in 5' von dem zu MPL2 identischen bzw. dem zu MPR2 komplementären Sequenzabschnitt einen Sequenzabschnitt PCL2 bzw. PCR2 aufweisen.

5. Das Verfahren gemäß einem der vorstehenden Ansprüche 1 bis 4, wobei eine zweite matrizenabhängige Nukleinsäurepolymerase, insbesondere eine DNA-Polymerase, sowie ggf. Substrate der matrizenabhängigen Nukleinsäurepolymerase (insbesondere Ribonukleosid-triphosphate oder Desoxyribonukleosid-triphosphate) und geeignete Kofaktoren), in dem zweiten Amplifikationsschritt mit der Probe in Kontakt gebracht wird.

6. Das Verfahren gemäß einem der vorstehenden Ansprüche 1 bis 5, **dadurch gekennzeichnet, dass** die modifizierten Nukleotidbausteine 2'-O-Alkylribonukleosidbausteine, insbesondere 2'-O-Methylribonukleosidbausteine, umfassen.

7. Das Verfahren nach einem der vorherigen Ansprüche 1 bis 6, **dadurch gekennzeichnet, dass** der erste Amplifikationsschritt im Wesentlichen isothermal durchgeführt wird.

8. Das Verfahren nach einem der vorherigen Ansprüche 1 bis 7, **dadurch gekennzeichnet, dass**
- MS eine Länge von 20 Nukleotiden bis 200 Nukleotiden hat; und/oder.
- PR1 eine Länge im Bereich von 15 Nukleotiden bis 100 Nukleotiden hat; und/oder
- PC eine Länge im Bereich von 5 Nukleotiden bis 85 Nukleotiden hat, insbesondere dass PCR zwischen 50% und 300% der Länge des Sequenzabschnitts PMR hat; und/oder
- C eine Länge im Bereich von 20 Nukleotiden bis 100 Nukleotiden hat.

9. Das Verfahren nach einem der vorherigen Ansprüche 1 bis 8, **dadurch gekennzeichnet, dass** die erste Amplifikation und die zweite Amplifikation im selben Reaktionsansatz durchgeführt werden.

10. Das Verfahren nach einem der vorherigen Ansprüche 1 bis 9, **dadurch gekennzeichnet, dass**
- die zweite matrizenabhängige Nukleinsäurepolymerase eine thermostabile Polymerase, insbesondere eine thermostabile DNA-Polymerase ist; und/oder
- die erste matrizenabhängige Nukleinsäurepolymerase thermisch inaktivierbar ist, insbesondere dass die erste matrizenabhängige Nukleinsäurepolymerase eine mesophile Polymerase ist.

11. Das Verfahren nach einem der vorherigen Ansprüche 1 bis10, **dadurch gekennzeichnet, dass** die zweite matrizenabhängige Nukleinsäurepolymerase, und / oder der zweite rechte Oligonukleotidprimer PR2 und/oder der zweite linke Oligonukleotidprimer PL2 aktivierbar sind, und insbesondere vor oder während des zweiten Amplifikationsschrittes aktiviert werden, und/oder das Controller-Oligonukleotid CR deaktivierbar sind, und insbesondere und vor oder während des zweiten Amplifikationsschrittes deaktiviert wird.

12. Das Verfahren nach einem der vorherigen Ansprüche 1 bis 11, **dadurch gekennzeichnet, dass** die Komponenten des zweiten Amplifikationsschrittes nach Durchführung des ersten Amplifikationschritts mit der Probe in Kontakt gebracht werden.

13. Das Verfahren nach einem der vorherigen Ansprüche 1 bis 12, **dadurch gekennzeichnet, dass** CR in CSR und /oder CPR einen Sequenzbereich umfasst, der sequenzspezifisch an den zweiten rechten Oligonukleotidprimer PR2 (komplementär) binden kann, wobei der Sequenzbereich insbesondere modifizierte Nukleotidanaloga umfasst, welche eine Verwendung von CR als Matrize verhindern.

14. Ein Kit zur Durchführung eines Verfahrens nach einem der Ansprüche 1 bis 13, umfassend:
- ein erster rechter Oligonukleotidprimer PR1, der in 5'-3'-Orientierung in unmittelbarer Folge die Sequenzabschnitte PCR und PMR umfasst, wobei PMR an eine Primerbindungsstelle MPR einer genomischen Sequenz M eines eukaryoten Organismus oder eines pathogenen Bakteriums, insbesondere eines Säugetiers, ganz inbesondere an eine menschliche Zielsequenz, binden kann, wobei M in 5'-3'-Orientierung in unmittelbarer Folge die Sequenzabschnitte MPL, MS und MPR umfasst, und der Sequenzabschnitt PCR nicht an eine direkt in 3' von MPR liegende Sequenz binden kann, und wobei
PR1 (insbesondere im Abschnitt PCR) modifizierte Nukleotidbausteine umfasst, so dass PCR nicht als Matrize für die Aktivität der ersten matrizenabhängigen Nukleinsäurepolymerase dienen kann; und
- ein erster linker Oligonukleotidprimer PL1, welcher (im Wesentlichen) identisch mit MPL ist;
- ein Controller-Oligonukleotid CR, das in 5'-3'-Orientierung die Sequenzabschnitte CSR, CPR und CCR in unmittelbarer Folge umfasst, wobei CSR identisch zu einem Abschnitt von MS ist, welcher in 5' von MPR liegt [und bei Initiation der Polymerase von PR1 zuerst abgelesen wird, CSR also an das Verlängerungsprodukt des Primers PR1 binden kann], CPR komplementär zu PMR (und identisch zu MPR) ist und CCR eine zu PCR komplementäre Sequnz umfasst, und
wobei CR in CSR und / oder CPR modifizierte Nukleotidbausteine umfasst, so dass CSR nicht als Matrize für die Aktivität der matrizenabhängigen Nukleinsäurepolymerase dienen kann;
- ein zweiter linker Oligonukleotidprimer PL2, welcher zu einem ersten Sekundärprimerbindungsbereich MPL2 identisch ist, wobei insbesondere zumindest das 3'-Segment von MPL2 innerhalb von MPL oder MS liegt, und
- ein zweiter rechter Oligonukleotidprimer PR2, welcher zu einem Bereich MPR2 komplementär ist, wobei insbesondere zumindest das 3'-Segment von MPR2 innerhalb von MS oder MPR liegt,
- wobei MPL2 und MPR2 von M umfasst werden und das 3'-Ende von MPL2 [mindestens 5, 10 oder 20 Positionen] in 5'-Richtung des 5'-Endes von MPR2 angeordnet ist, (insbesondere MPL2 mit MPL und /oder MPR2 mit MPR identisch ist).

15. Der Kit nach der Anspruch 14, weiterhin umfassend
- eine erste matrizenabhängige Nukleinsäurepolymerase, insbesondere eine DNA-Polymerase, sowie optional Substrate der DNA-Polymerase (insbesondere Ribonukleosid-triphosphate oder Desoxyribonukleosid-triphosphate) und geeignete Kofaktoren, insbesondere eine mesophile matritzenabhängige Polymerase, insbesondere eine mesophile matritzenabhängige Polymerase ohne 5'-3'-Exonukleaseaktivität; und/oder
- eine zweite matrizenabhängige Nukleinsäurepolymerase, insbesondere eine DNA-Polymerase, sowie optional Substrate der matrizenabhängigen Nukleinsäurepolymerase (insbesondere Ribonukleosid-triphosphate oder Desoxyribonukleosid-triphosphate) und geeignete Kofaktoren, insbesondere eine thermophile matritzenabhängige Polymerase, insbesondere eine thermophile matritzenabhängige Polymerase mit 5'-3'-Exonukleaseaktivität.
